# FASCICULE DE BREVET EUROPEEN

(11) **EP 1 411 057 B1**
(45) Date de publication et mention de la délivrance du brevet: **27.04.2005**
(21) Numéro de dépôt: 03292545.5
(22) Date de dépôt: 14.10.2003
(51) Int. Cl.: C07D 487/14, C07D 491/14, C07D 471/22, A61K 31/40, A61P 35/00, C07H 19/23

(54) **Dérivés de [3, 4 - a:3, 4-c] carbazole, leur procédé de préparation et les compositions pharmaceutiques qui les contiennent**
[3,4-a:3,4-c]Carbazolderivate, Verfahren zu ihrer Herstellung und diese enthaltende pharmazeutische Zubereitungen
[3,4-a:3,4-c]carbazole derivatives, process for their preparation and pharmaceutical compositions containing them

(30) Priorité: 16.10.2002 FR 0212847
(43) Date de publication de la demande: 21.04.2004
(73) Titulaire: Les Laboratoires Servier, 92415 Courbevoie Cedex (FR)
(72) Inventeur: Prudhomme, Michelle, 63000 Clermont-Ferrand (FR); Hugon, Bernadette, 63000 Clermont-Ferrand (FR); Anizon, Fabrice, 63720 Ennezat (FR); Hickman, John, 75017 Paris (FR); Pierre, Alain, 78580 Les Alluets le Roi (FR); Golsteyn, Roy, 78780 Maurecourt (FR); Renard, Pierre, 78150 Le Chesnay (FR); Pfeiffer, Bruno, 95320 Saint Leu La Foret (FR)

(56) Documents cités:
- EP-A- 0 388 956

## Description

La présente invention concerne de nouveaux dérivés de [3,4-a:3,4-c]carbazole, leur procédé de préparation et les compositions pharmaceutiques qui les contiennent.

Les besoins de la thérapeutique anticancéreuse exigent le développement constant de nouveaux agents antiprolifératifs, dans le but d'obtenir à la fois des médicaments plus actifs et mieux tolérés. Les composés de la présente invention présentent notamment des propriétés anti-tumorales, les rendant ainsi utiles dans le traitement des cancers. Parmi les types de cancers qui peuvent être traités par les composés de la présente invention, on peut citer à titre non limitatif les adénocarninomes et carcinomes, sarcomes, gliomes et leucémies. De part leur propriétés, les composés de l'invention peuvent être associés avantageusement à l'ensemble des traitements cytotoxiques actuellement en usage, mais aussi aux radiothérapies, dont ils n'augmentent pas la toxicité, et aux divers hormonothérapies à visée anti-cancéreuses (sein et prostate).

Les demandes de brevets WO 95/07910 et WO 96/04906 décrivent des dérivés d'indole et les revendiquent d'une part pour leur activité antivirale et d'autre part pour le traitement et la prévention de la resténose. Les demandes de brevet WO 00/47583, WO 97/21677 et WO 96/11933 présentent des dérivés de cyclopenta[g]pyrrolo[3,4-*e*]indole fusionnés par la partie indole et la partie cyclopentène des dérivés, à un système cyclique aromatique ou non aromatique, et comportant éventuellement des hétéroatomes. Ces composé possèdent des activités pharmacologiques les rendant notamment utiles dans le traitement du cancer. La demande de brevet WO 01/85686 décrit des dérivés de pyrrolo[3,4-c] carbazole utiles dans le traitement des maladies neurodégénératives, des inflammations, de l'ischémie et du cancer. La demande de brevet WO 02/24699 décrit des dérivés tétrahydrocarbazole utiles d'une part dans le traitement antimicrobien et d'autre part en tant que désodorisant et désinfectant de la peau.

Plus particulièrement, la présente invention concerne les composés de formule (I) : 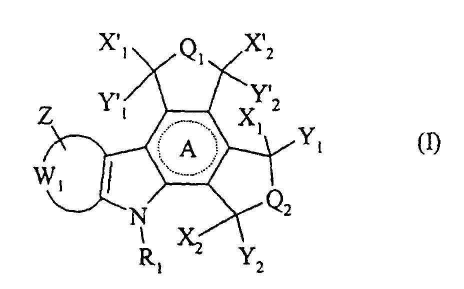 dans laquelle :
- **A** représente un cycle saturé, partiellement ou totalement insaturé pouvant éventuellement conférer un caractère aromatique au cycle,
- **W**_{**t**} représente, avec les atomes de carbone auxquels il est lié, un groupement phényle ou un groupement pyridinyle,
- **Z** représente un ou plusieurs groupements identiques ou différentes de formule U-V dans laquelle :
   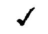 U représente une liaison simple, une chaîne alkylène (C₁-C₆) linéaire ou ramifiée ou une chaîne alkényle (C₂-C₆) linéaire ou ramifiée, éventuellement substituée par un ou plusieurs groupements, identiques ou différents, choisis parmi halogène et hydroxy, et/ou contenant éventuellement une ou plusieurs insaturations,
   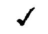 V représente un groupement choisi parmi atome d'hydrogène, d'halogène, groupement cyano, nitro, azido, alkyle (C₁-C₆) linéaire ou ramifié, aryle, arylalkyle (C₁-C₆) linéaire ou ramifié, hydroxy, alkoxy (C₁-C₆) linéaire ou ramifié, aryloxy, arylalkoxy (C₁-C₆) linéaire ou ramifié, formyle, carboxy, aminocarbonyle, NR₃R₄, -C(O)-T₁, -C(O)-NR₃-T₁, -NR₃-C(O)-T₁, -O-C(O)-T₁, -C(O)-O-T₁, -NR₃-T₂-NR₃R₄, -NR₃-T₂-OR₃, -NR₃-T₂-CO₂R₃, -O-T'₂-NR₃R₄, -O-T'₂-OR₃, -O-T'₂-CO₂R₃, et -S(O)ₜ-R₃,
   dans lesquels :
   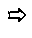 R₃ et R₄, identiques ou différents, représentent chacun un groupement choisi parmi atome d'hydrogène, groupement alkyle (C₁-C₆) linéaire ou ramifié, aryle, et arylalkyle (C₁-C₆) linéaire ou ramifié, ou
      R₃+R₄ forment ensemble, avec l'atome d'azote qui les portent, un hétérocycle de 5 à 10 atomes, monocyclique ou bicyclique, saturé, contenant éventuellement au sein du système cyclique un second hétéroatome choisi parmi oxygène et azote, et étant éventuellement substitué par un groupement choisi parmi alkyle (C₁-C₆) linéaire ou ramifié, aryle, arylalkyle (C₁-C₆) linéaire ou ramifié, hydroxy, alkoxy (C₁-C₆) linéaire ou ramifié, amino, monoalkylamino (C₁-C₆) linéaire ou ramifié, et dialkylamino (C₁-C₆) linéaire ou ramifié,
   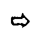 T₁ représente un groupement choisi parmi alkyle (C₁-C₆) linéaire ou ramifié, éventuellement substituée par un groupement choisi parmi -OR₃, -NR₃R₄, -CO₂R₃, -C(O)R₃ et -C(O)NR₃R₄ dans lesquels R₃ et R₄ sont tels que définis précédemment, aryle, arylalkyle (C₁-C₆) linéaire ou ramifié, ou T₁ représente une chaîne alkényle (C₂-C₆) linéaire ou ramifiée éventuellement substituée par un groupement choisi parmi -OR₃, -NR₃R₄, -CO₂R₃, -C(O)R₃ et -C(O)NR₃R₄ dans lesquels R₃ et R₄ sont tels que définis précédemment,
   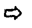 T₂ représente une chaîne alkylène (C₁-C₆) linéaire ou ramifiée,
   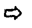 T'₂ représente une chaîne alkylène (C₁-C₆) linéaire ou ramifiée éventuellement substituée par un ou plusieurs groupements hydroxy,
   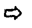 t représente un entier compris entre 0 et 2 inclus,
   ou Z représente un groupement méthylènedioxy ou un groupement éthylènedioxy,
- **Q**_{**1**} représente un groupement choisi parmi atome d'oxygène ou un groupement NR₂ dans lequel R₂ représente un groupement choisi parmi atome d'hydrogène, groupement alkyle (C₁-C₆) linéaire ou ramifié, aryle, arylalkyle (C₁-C₆) linéaire ou ramifié, cycloalkyle, cycloalkylalkyle (C₁-C₆) linéaire ou ramifié, -OR₃, -NR₃R₄, -O-T₂-NR₃R₄, -NR₃-T₂-NR₃R₄, hydroxyalkylamino (C₁-C₆) linéaire ou ramifié, di(hydroxyalkyl) amino (C₁-C₆) linéaire ou ramifié, -C(O)-R₃, -NH-C(O)-R₃, ou une chaîne alkylène (C₁-C₆) linéaire ou ramifié substituée par un ou plusieurs groupements, identiques ou différents, choisis parmi atomes d'halogène, groupements cyano, nitro, -OR₃, -NR₃R₄, -CO₂R₃, -C(O)R₃, hydroxyalkylamino (C₁-C₆) linéaire ou ramifié, di(hydroxyalkyl)amino (C₁-C₆) linéaire ou ramifié, et -C(O)-NHR₃, les groupements R₃, R₄ et T₂ ayant les mêmes significations que précédemment,
- **Q**_{**2**} représente un groupement choisi parmi atome d'oxygène ou un groupement NR'₂ dans lequel R'₂ représente un groupement choisi parmi atome d'hydrogène, groupement alkyle (C₁-C₆) linéaire ou ramifié, aryle, arylalkyle (C₁-C₆) linéaire ou ramifié, cycloalkyle, cycloalkylalkyle (C₁-C₆) linéaire ou ramifié, -OR₃, -NR₃R₄, -O-T₂-NR₃R₄, -NR₃-T₂-NR₃R₄, hydroxyalkylamino (C₁-C₆) linéaire ou ramifié, di(hydroxyalkyl)amino (C₁-C₆) linéaire ou ramifié, -C(O)-R₃, NH-C(O)-R₃, ou une chaîne alkylène (C₁-C₆) linéaire ou ramifié substituée par un ou plusieurs groupements, identiques ou différents, choisis parmi atomes d'halogène, groupements cyano, nitro, -OR₃, -NR₃R₄, -CO₂R₃, -C(O)R₃, hydroxyalkylamino (C₁-C₆) linéaire ou ramifié, di(hydroxyalkyl)amino (C₁-C₆) linéaire ou ramifié, et -C(O)-NHR₃, les groupements R₃, R₄ et T₂ ayant les mêmes significations que précédemment,

- **X**_{**1**} représente un groupement choisi parmi atome d'hydrogène, groupement hydroxy, alkoxy (C₁-C₆) linéaire ou ramifié, mercapto, et alkylthio (C₁-C₆) linéaire ou ramifié,
- **Y**_{**1**} représente un atome d'hydrogène, ou
- **X**_{**1**} et **Y**_{**1**} forment ensemble, avec l'atome de carbone qui les porte, un groupement carbonyle ou thiocarbonyle,

- **X**_{**2**} représente un groupement choisi parmi atome d'hydrogène, groupement hydroxy, alkoxy (C₁-C₆) linéaire ou ramifié, mercapto, et alkylthio (C₁-C₆) linéaire ou ramifié,
- **Y**_{**2**} représente un atome d'hydrogène, ou
- **X**_{**2**} et **Y**_{**2**} forment ensemble, avec l'atome de carbone qui les porte, un groupement carbonyle ou thiocarbonyle,

- **X'**_{**1**} représente un groupement choisi parmi atome d'hydrogène, groupement hydroxy, alkoxy (C₁-C₆) linéaire ou ramifié, mercapto, et alkylthio (C₁-C₆) linéaire ou ramifié,
- **Y'**_{**1**} représente un atome d'hydrogène, ou
- **X'**_{**1**} et **Y'**_{**1**} forment ensemble, avec l'atome de carbone qui les porte, un groupement carbonyle ou thiocarbonyle,

- **X'**_{**2**} représente un groupement choisi parmi atome d'hydrogène, groupement hydroxy, alkoxy (C₁-C₆) linéaire ou ramifié, mercapto, et alkylthio (C₁-C₆) linéaire ou ramifié,
- **Y'**_{**2**} représente un atome d'hydrogène, ou
- **X'**_{**2**} et **Y'**_{**2**} forment ensemble, avec l'atome de carbone qui les porte, un groupement carbonyle ou thiocarbonyle,

- **R**_{**1**} représente un groupement choisi parmi atome d'hydrogène, groupement alkyle (C₁-C₆) linéaire ou ramifié éventuellement substitué par un ou plusieurs groupements hydroxy, alkoxy (C₁-C₆) linéaire ou ramifié, hydroxyalkoxy (C₁-C₆) linéaire ou ramifié, NR₃R₄, les groupements R₃ et R₄ ayant les mêmes définitions que précédemment, ou R₁ représente un groupement de formule (a) : 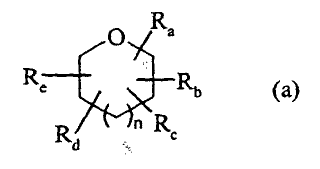 dans laquelle :
   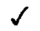 **R**_{**a**}**, R**_{**b**}**, R**_{**c**} **et R**_{**d**} identiques ou différents, indépendamment l'un de l'autre, représentent chacun une liaison ou un groupement choisi parmi atome d'hydrogène, atome d'halogène, groupement hydroxy, alkoxy (C₁-C₆) linéaire ou ramifié, aryloxy, arylalkoxy (C₁-C₆) linéaire ou ramifié, alkyle (C₁-C₆) linéaire ou ramifié, arylalkyle (C₁-C₆) linéaire ou ramifié, aryle, -NR₃R₄ dans lequel R₃ et R₄ sont tels que définis précédemment, azido, -N=NR₃ (dans lequel R₃ est tel que défini précédemment), et -O-C(O)-R₅ dans lequel R5 représente un groupement alkyle (C₁-C₆) linéaire ou ramifié (éventuellement substitué par un ou plusieurs groupements choisis parmi halogène, hydroxy, amino, alkylamino (C₁-C₆) linéaire ou ramifié, et dialkylamino (C₁-C₆) linéaire ou ramifié), aryle, arylalkyle (C₁-C₆) linéaire ou ramifié, cycloalkyle, ou hétérocycloalkyle,
   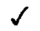 **R**_{**e**} représente un groupement méthylène (H₂C=) ou un groupement de formule -U₁-Rₐ dans laquelle U₁ représente une liaison simple ou un groupement méthylène, et Rₐ est tel que défini précédemment,
   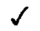 **n** prend la valeur 0 ou 1,
étant entendu que le groupement de formule (a) est lié à l'atome d'azote par Rₐ, R_{b}, R_{c}, R_{d} ou Rₑ,
leurs énantiomères, diastéréoisomères, ainsi que leurs sels d'addition à un acide ou à une base pharmaceutiquement acceptable,
étant entendu que les composés de formule (I) sont différents des composés suivants :
- 3b,6a,6b,7-tétrahydro-1*H*-dipyrrolo[3,4-a:3,4-c]carbazole-1,3,4,6(2*H*,3a*H*,5*H*)-tétrone ;
- 5-éthyl-3b,6a,6b,7-tétrahydro-1*H*-dipyrrolo[3,4-a:3,4-c]carbazole-1,3,4,6(2*H*,3a*H*,5*H*)-tétrone ;
- 3b,6a,7,11c-tétrahydro-1*H*-dipyrrolo[3,4-a:3,4-c]carbazole-1,3,4,6(2*H*,3a*H*,5*H*)-tétrone ;
- 3b,6a,6b,7-tétrahydrofuro[3,4-a]pyrrolo[3,4-c]carbazole-1,3,4,6(2*H*,3a*H*,5*H*)-tétrone ;
étant entendu que par aryle, on comprend un groupement phényle, naphtyle, dihydronaphtyle, tétrahydronaphtyle, indényle ou indanyle, chacun de ces groupements étant éventuellement substitué par un ou plusieurs groupements, identiques ou différents, choisis parmi halogène, alkyle (C₁-C₆) linéaire ou ramifié, trihalogénoalkyle (C₁-C₆) linéaire ou ramifié, hydroxy, alkoxy (C₁-C₆) linéaire ou ramifié, et NR₃R₄, R₃ et R₄ ayant les mêmes définitions que précédemment.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

Les composés préférés de l'invention sont ceux pour lesquels X₁ et Y₁ forment ensemble avec l'atome de carbone qui le porte, un groupement carbonyle, X₂ et Y₂ forment ensemble avec l'atome de carbone qui le porte, un groupement carbonyle, X'₁ et Y'₁ forment ensemble avec l'atome de carbone qui le porte, un groupement carbonyle et X'₂ et Y'₂ forment ensemble avec l'atome de carbone qui le porte, un groupement carbonyle.

D'une façon avantageuse, le groupement Q₁ préféré selon l'invention est le groupement -NR₂ dans lequel R₂ est tel que défini dans la formule (I).

D'une façon avantageuse, le groupement Q₂ préféré selon l'invention est le groupement -NR'₂ dans lequel R'₂ est tel que défini dans la formule (I).

Selon une variante avantageuse, les composés préférés de l'invention sont les composés de formule (I) répondant plus particulièrement à la formule (IA) : 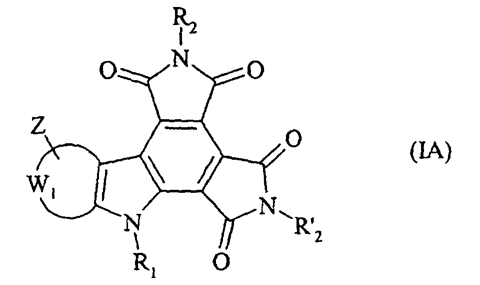 dans laquelle R₁, R₂, R'₂, W₁ et Z sont tels que définis dans la formule (I).

Selon une deuxième variante avantageuse, les composés préférés de l'invention sont les composés de formule (I) répondant plus particulièrement à la formule (IB) : 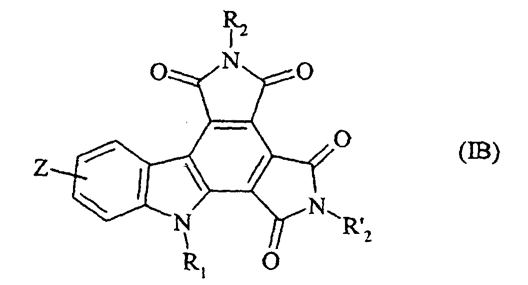 dans laquelle R₁, R₂, R'₂ et Z sont tels que définis dans la formule (I).

Selon une troisième variante avantageuse, les composés préférés de l'invention sont les composés de formule (I) répondant plus particulièrement à la formule (IC) : 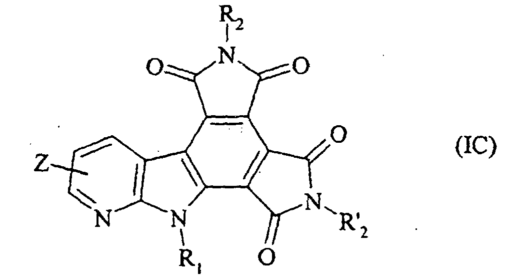 dans laquelle R₁, R₂, R'₂ et Z sont tels que définis dans la formule (I).

Selon une quatrième variante avantageuse, les composés préférés de l'invention sont les composés de formule (I) répondant plus particulièrement à la formule (ID) : 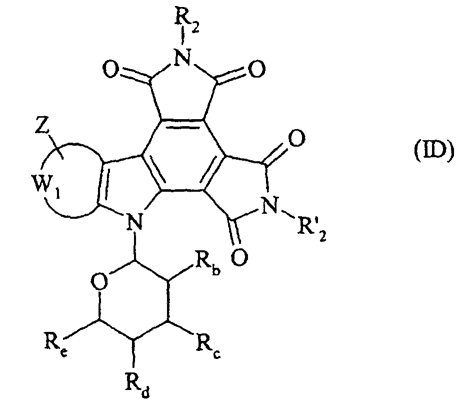 dans laquelle R₂, R'₂, W₁, Z, R_{b}, R_{c}, R_{d} et Rₑ sont tels que définis dans la formule (I).

Selon une cinquième variante avantageuse, les composés préférés de l'invention sont les composés de formule (I) répondant plus particulièrement à la formule (IE) : 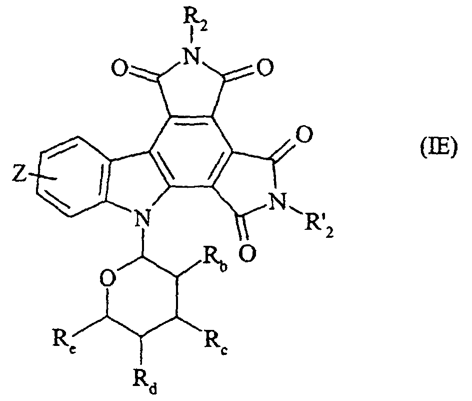 dans laquelle R₂, R'₂, Z, R_{b}, R_{c}, R_{d} et Rₑ sont tels que définis dans la formule (I).

Selon une sixième variante avantageuse, les composés préférés de l'invention sont les composés de formule (I) répondant plus particulièrement à la formule (IF) : 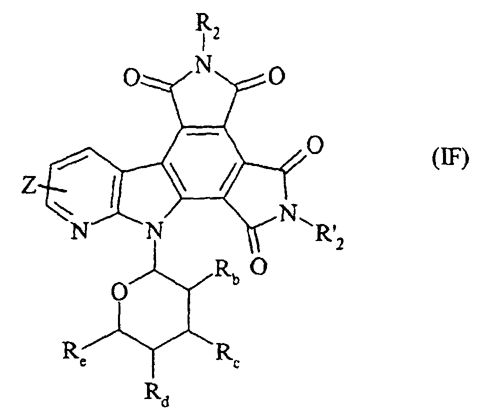 dans laquelle R₂, R'₂, Z, R_{b}, R_{c}, R_{d} et Rₑ sont tels que définis dans la formule (I).

D'une façon préférentielle, le substituant Z préféré selon l'invention est le groupement de formule U-V dans laquelle U représente une liaison simple et V représente un groupement choisi parmi atome d'hydrogène, d'halogène, groupement nitro, alkyle (C₁-C₆) linéaire ou ramifié, hydroxy, alkoxy (C₁-C₆) linéaire ou ramifié, arylalkoxy (C₁-C₆) linéaire ou ramifié et NR₃R₄ dans lequel R₃ et R₄ représentent chacun un atome d'hydrogène.

D'une façon encore plus préférentielle, le substituent Z préféré selon l'invention est le groupement de formule U-V dans laquelle U représente une liaison simple et V représente un groupement choisi parmi atome d'hydrogène, halogène, groupement hydroxy et arylalkoxy (C₁-C₆) linéaire ou ramifié.

D'une façon intéressante, le groupement R₁ préféré selon l'invention est l'atome d'hydrogène, le groupement alkyle (C₁-C₆) linéaire ou ramifié et le groupement de formule (a) : 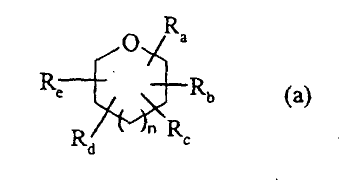 lié à l'atome d'azote par Ra
dans laquelle :
- R_{b}, R_{c}, et R_{d} représentent un groupement hydroxy, arylalkoxy (C₁-C₆) linéaire ou ramifié -O-C(O)-R₅ dans lequel R₅ représente un groupement alkyle (C₁-C₆) linéaire ou ramifié,
- Rₑ représente un groupement de formule U₁-Rₐ dans laquelle U₁ représente un groupement méthylène et Rₐ prend les mêmes définitions que R_{b}, R_{c} et R_{d} et n prend la valeur 0.

D'une façon encore plus intéressante, le groupement R₁ préféré selon l'invention est l'atome d'hydrogène.

D'une façon intéressante, le groupement R₂ préféré selon l'invention sont l'atome d'hydrogène, le groupement alkyle (C₁-C₆) linéaire ou ramifié, OR₃, NR₃R₄ et une chaîne alkylène (C₁-C₆) linéaire ou ramifié substituée par un groupement OR₃ ou NR₃R₄ dans lequels R₃ et R₄ sont tels que définis dans la formule (I).

D'une façon encore plus intéressante, le groupement R₂ préférés selon l'invention sont l'atome d'hydrogène, le groupement alkyle (C₁-C₆) linéaire ou ramifié et une chaîne alkylène (C₁-C₆) linéaire ou ramifié substituée par un groupement NR₃R₄ dans lequel R₃ et R₄ sont tels que définis dans la formule I.

D'une façon intéressante, les groupements R'₂ préférés selon l'invention sont l'atome d'hydrogène, le groupement alkyle (C₁-C₆) linéaire ou ramifié, et une chaîne alkylène (C₁-C₆) linéaire ou ramifié substituée par un groupement NR₃R₄ dans lequel R₃ et R₄ sont tels que définis dans la formule (I).

D'une façon intéressante, le groupement de formule (a) préféré selon l'invention est le groupement glucopyranosyl de formule :

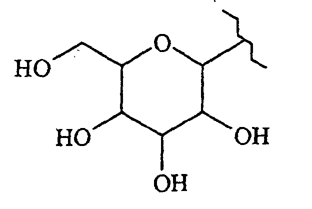

Les composés préférés selon l'invention sont le :
- 1*H*-dipyrrolo[3,4-a:3,4-c]carbazole-1,3,4,6(2*H*,5*H*,7*H*)-tétrone,
- 2-méthyl-1*H*-dipyrrolo[3,4-a:3,4-c]carbazole-1,3,4,6(2*H*,5*H*,7*H*)-tétrone,
- 2,5-diméthyl-1*H*-dipyrrolo[3,4-a:3,4-c]carbazole-1,3,4,6(2*H*,5*H*,7*H*)-tétrone,
- 2-[2-(diéthylamino)éthyl]-5-méthyl-1*H*-dipyrrolo[3,4-a:3,4-c]carbazole-1,3,4,6 (2*H*,5*H*,7*H*)-tétrone,
- 10-hydroxy-1*H*-dipyrrolo[3,4-a:3,4-c]carbazole-1,3,4,6(2*H*,5*H*,7*H*)-tétrone.

Les énantiomères, diastéréoisomères ainsi que les sels d'addition à un acide ou à une base pharmaceutiquement acceptable des composés préférés font partie intégrante de l'invention.

La présente invention concerne également le procédé de préparation des composés de formule (I), caractérisé en ce qu'on utilise comme produit de départ un composé de formule (II) : 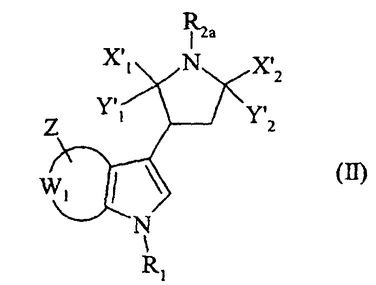 dans laquelle R₂ₐ représente un atome d'hydrogène ou un groupement méthyle et, R₁, X'₁, Y'₁, X'₂, Y'₂, W₁ et Z sont tels que définis dans la formule (I),
qui est traité avec du 2,3-dichloro-5,6-dicyano-1,4-benzoquinone pour conduire au composé de formule (III) : 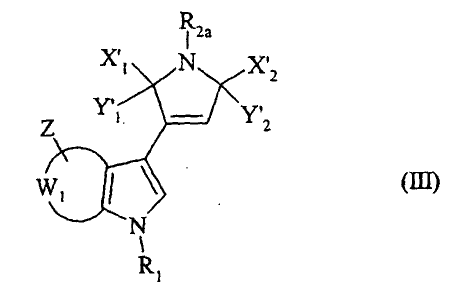 dans laquelle R₁, R₂ₐ, X'₁, Y'₁, X'₂, Y'₂, W₁ et Z sont tels que définis précédemment, composé de formule (III) qui est :
* *soit* traité par de la soude aqueuse puis placé en présence d'acide chlorhydrique pour conduire au composé de formule (IV) : 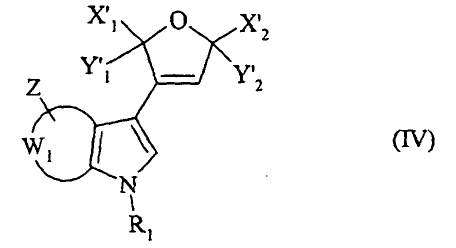
dans laquelle R₁, X'₁, Y'₁, X'₂, Y'₂, W₁ et Z sont tels que définis précédemment, composé de formule (IV) qui est traité par un composé de formule (V) : 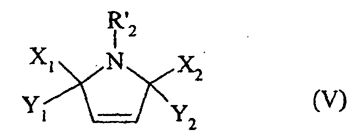 dans laquelle R'₂, X₁, Y₁, X₂ et Y₂ sont tels que définis dans la formule (I) pour conduire au composé de formules (Va) et (I/b), cas particulier des composés de formule (I) : 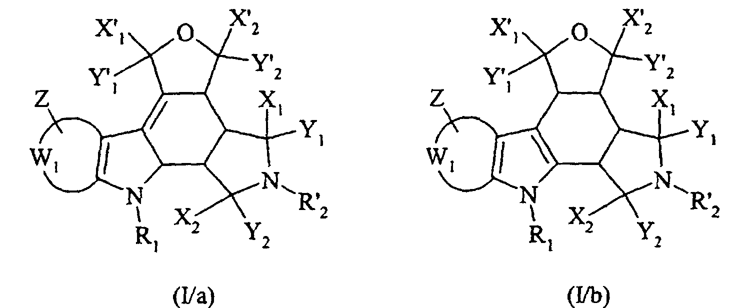 dans laquelle R₁, R'₂, X₁, Y₁, X₂, Y₂, X'₁, Y'₁, X'₂, Y'₂, W₁ et Z sont tels que définis précédemment,
composés de formule (I/a) et/ou (I/b) qui sont éventuellement soumis à l'action d'acide trifluoroacétique pour conduire au composé de formule (I/c), cas particulier des composés de formule (I) : 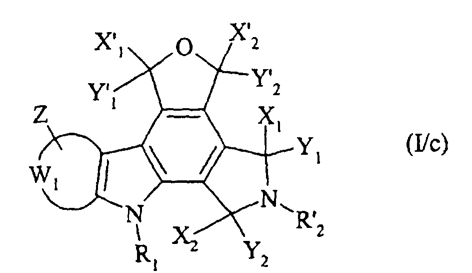 dans laquelle R₁, R'₂, X₁, Y₁, X₂, Y₂, X'₁, Y'₁, X'₂, Y'₂, W₁ et Z sont tels que définis précédemment,
l'ensemble des composés de formules (I/a), (I/b) et (I/c) forment les composés de formule (I/d) : 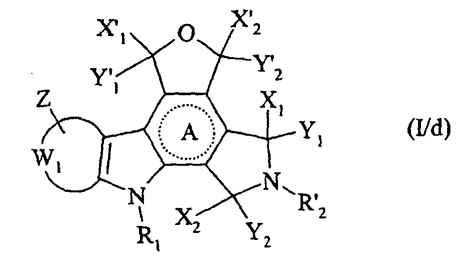 dans laquelle A, R₁, R'₂, X₁, Y₁, X₂, Y₂, X'₁, Y'₁, X'₂, Y'₂, W₁ et Z sont tels que définis précédemment,
composé de formule (I/d) qui est éventuellement soumis à l'action d'un composé de formule (VII) :

R_{2b} - NH₂ (VII)

dans laquelle R_{2b} a la même définition que R₂, dans la formule (I), à l'exception des définitions atome d'hydrogène et groupement méthyle, pour conduire aux composés de formule (I/e), cas particulier des composés de formule (I) : 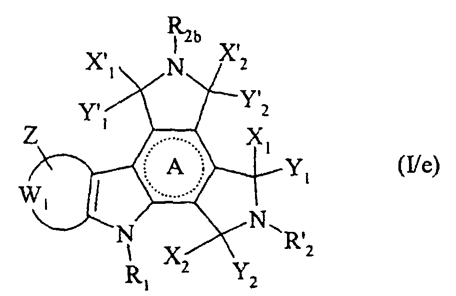 dans laquelle A, R₁, R'₂, R_{2b}, X₁, Y₁, X₂, Y₂, X'₁, Y'₁, X'₂, Y'₂, W₁ et Z sont tels que définis précédemment,
* *soit* soumis successivement aux mêmes conditions de réaction que les composés de formules (IV), (I/a) et (I/b), pour conduire au composé de formule (I/f), cas particulier des composés de formule (I) : 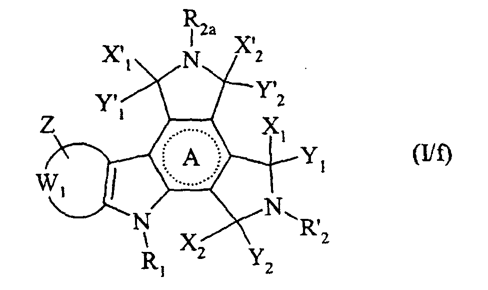 dans laquelle A, R₁, R'₂, R₂ₐ, X₁, Y₁, X₂, Y₂, X'₁, Y'₁, X'₂, Y'₂, W₁ et Z sont tels que définis précédemment,
   l'ensemble des composés (I/d), (I/e) et (I/f) formant les composés de formule (I/g) : 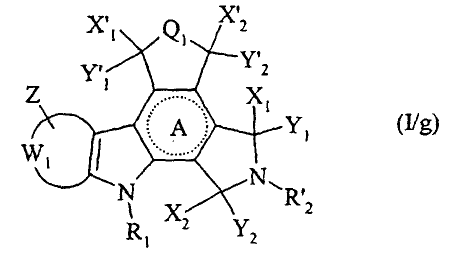 dans laquelle A, R₁, R'₂, Q₁, X₁, Y₁, X₂, Y₂, X'₁, Y'₁, X'₂, Y'₂, W₁ et Z sont tels que définis précédemment,
   composé de formule (I/g) qui, dans le cas particulier où R'₂ représente un atome d'hydrogène ou un groupement méthyle, est éventuellement soumis successivement aux mêmes conditions de réaction que le composé de formule (III) pour conduire au composé de formule (I/i), cas particulier des composés de formule (I) : 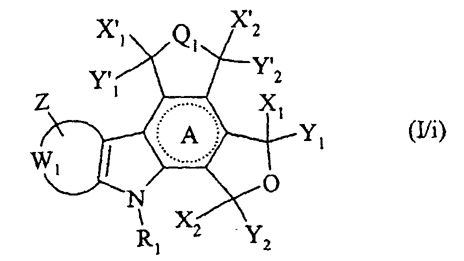 dans laquelle A, R₁, Q₁, X₁, Y₁, X₂, Y₂, X'₁, Y'₁, X'₂, Y'₂, W₁ et Z sont tels que définis précédemment,
   composé de formule (I/i) qui est éventuellement soumis à l'action d'un composé (VIII) :

   R'_{2b} - NH₂ (VIII)

   dans laquelle R'_{2b} a la même définition que R'₂ dans la formule (I), à l'exception des définitions atome d'hydrogène et groupement méthyle, pour conduire aux composés de formule (I/j), cas particulier des composés de formule (I) : 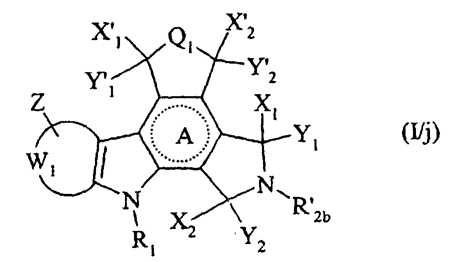 dans laquelle A, R₁, R'_{2b}, Q₁, X₁, Y₁, X₂, Y₂, X'₁, Y'₁, X'₂, Y'₂, W₁ et Z sont tels que définis précédemment,
   les composés de formule (I/a) à (I/j) formant l'ensemble des composés de formule (I), que l'on purifie, le cas échéant, selon des techniques classiques de purification, qui peuvent, si on le désire, être séparés en leurs différents isomères, selon une technique classique de séparation, dont on module les substituants Rₐ, R_{b}, R_{c}, R_{d} et Rₑ selon les méthodes classiques de la synthèse organique utilisées dans le domaine de la chimie des sucres, et que l'on transforme, si on le souhaite, en leurs sels d'addition à un acide ou à une base pharmaceutiquement acceptable.
   Les composés de formule (II) peuvent avantageusement être obtenus à partir d'un composé de formule (A) : 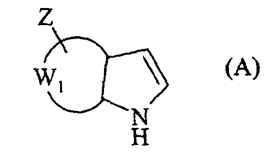 dans laquelle W₁ et Z sont tels que définis dans la formule (I), que l'on fait réagir :
* *soit* avec un composé de formule (B) : 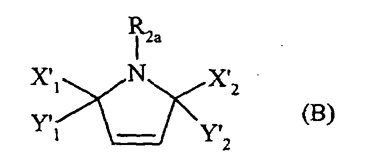 dans laquelle R₂ₐ, X'₁, Y'₁, X'₂ et Y'₂ sont tels que définis précédemment, pour conduire au composé de formule (C) : 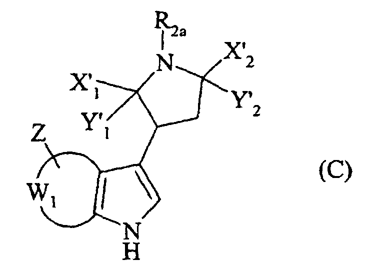 dans laquelle R₂ₐ, X'₁, Y'₁, X'₂, Y'₂, W₁ et Z sont tels que définis précédemment,
   composé de formule (C) qui éventuellement est soumis à l'action d'un composé de formule (IX):

   R₁ₐ - G (IX)

   dans laquelle G représente un groupement hydroxy ou un groupement partant et R₁ₐ, différent d'un atome d'hydrogène, a la même définition que R₁ dans la formule (I),
   pour conduire au composé de formule (D) : 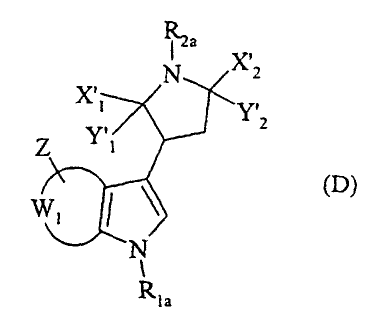 dans laquelle R₁ₐ, R₂ₐ, X'₁, Y'₁, X'₂, Y'₂, W₁ et Z sont tels que définis précédemment,
   les composés de formules (C) et (D) formant l'ensemble des composés de formule (II),
* *soit* avec un composé de formule (E), en présence d'halogénure d'alkylmagnésium : 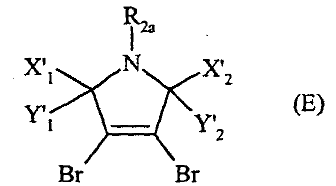 dans laquelle R₂ₐ, X'₁, Y'₁, X'₂ et Y'₂ sont tels que définis précédemment,
   pour conduire au composé de formule (F) : 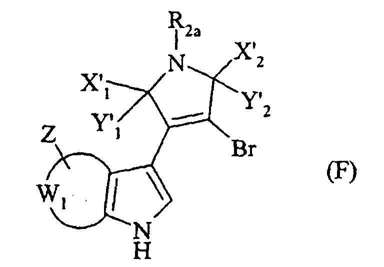 dans laquelle R₁ₐ, R₂ₐ, X'₁, Y'₁, X'₂, Y'₂, W₁ et Z sont tels que définis précédemment,
   composé de formule (F) qui est éventuellement soumis aux mêmes conditions de réaction que le composé de formule (C), pour conduire au composé de formule (G) : 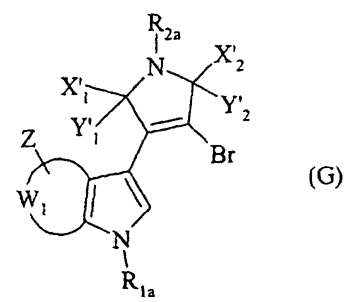 dans laquelle R₁ₐ, R₂ₐ, X'₁, Y'₁, X'₂, Y'₂, W₁ et Z sont tels que définis précédemment, composé de formule (G) qui est hydrogéné selon les méthodes classiques de la synthèse organique pour conduire au composé de formule (II).

Les composés de formule (V), (VII), (VIII), (IX), (A), (B) et (E) sont soit des produits commerciaux, soit obtenus selon des méthodes classiques de la synthèse organique bien connues de l'homme du métier.

Les composés de formule (I) présentent des propriétés anti-tumorales particulièrement intéressantes. Les propriétés caractéristiques de ces composés permettent leur utilisation en thérapeutique en tant qu'agents antitumoraux.

Les composés de l'invention peuvent également être utilisés en association thérapeutique avec un autre anticancéreux tel que, par exemple, le paclitaxel, le tamoxifène et ses dérivés, les cisplatines et ses analogues, l'irinotécan et ses métabolites, les divers alkylants dont le chef de file est le cyclophosphamide, l'étoposide, les vincaalcaloïdes, la doxorubicine et autres anthracyclines, les nitrosourées.

La présente invention a également pour objet les compositions pharmaceutiques contenant comme principe actif au moins un composé de formule (I), ses isomères optiques, ou un de ses sels d'addition à un acide ou à une base pharmaceutiquement acceptable, seul ou en combinaison avec un ou plusieurs excipients ou véhicules inertes, non toxiques, pharmaceutiquement acceptables.

Parmi les compositions pharmaceutiques selon l'invention, il sera cité plus particulièrement celles qui conviennent pour l'administration orale, parentérale (intraveineuse, intramusculaire ou sous-cutanée), per ou transcutanée, nasale, rectale, perlinguale, oculaire ou respiratoire, et notamment les comprimés simples ou dragéifiés, les comprimés sublinguaux, les gélules, les capsules, les suppositoires, les crèmes, les pommades, les gels dermiques, les préparations injectables ou buvables, les aérosols, les gouttes oculaires ou nasales, etc...

De part les propriétés pharmacologiques caractéristiques des composés de formule (I), les compositions pharmaceutiques contenant comme principe actif lesdits composés de formule (I), sont donc particulièrement utiles pour le traitement des cancers.

La posologie utile varie selon l'âge et le poids du patient, la voie d'administration, la nature et la sévérité de l'affection, et la prise de traitements éventuels associés et s'échelonne de 1 mg à 500 mg en une ou plusieurs prises par jour.

Les exemples suivant illustrent l'invention mais ne la limitent en aucune façon. Les produits de départs utilisés sont des produits connus ou préparés selon des modes opératoires connus.

Les structures des composés décrits dans les exemples ont été déterminées selon les techniques spectrophotométriques usuelles (infrarouge, résonance magnétique nucléaire, spectrométrie de masse, ...).

### PREPARATION A :

### 3b,6a,6b,7-tétrahydro-1H-dipyrrolo[3,4-a:3,4-c]carbazole-1,3,4,6(2H,3aH,5H)-tétrone

### Stade A : 3-(1H-indol-3-yl)-2,5-pyrrolidinedione

Le produit attendu est obtenu selon le procédé décrit par J. Bergman et coll. (Tetrahedron, 1999, 55, pp. 2363-2370).

### Stade B : 3-(1H-indol-3-yl)-1H-pyrrole-2,5-dione

Le produit attendu est obtenu selon le procédé décrit par J. Bergman et coll. (Tetrahedron, 1999, 55, pp. 2363-2370).

### Stade C : 3b,6a,6b,7-tétrahydro-1H-dipyrrolo[3,4-a:3,4-c]carbazole-1,3,4,6 (2H,3aH,5H)-tétrone

Le produit attendu est obtenu selon le procédé décrit par J. Bergman et coll. (J. Chem. Soc., Perkin Trans. I, 2000, pp. 2615-2621).

### PREPARATION B :

### 3-(1H-indol-3-yl)-1-méthyl-1H-pyrrole-2,5-dione

### Stade A : 3-(1H-indol-3-yl)-1-méthyl-2,5-pyrrolidinedione

Le produit attendu est obtenu selon le procédé décrit par J. Bergman et coll. (Tetrahedron, 1999, 55, pp. 2363-2370).

### Stade B : 3-(1H-indol-3-yl)-1-méthyl-1H-pyrrole-2,5-dione

Le produit attendu est obtenu selon le procédé décrit par J. Bergman et coll. (Tetrahedron, 1999, 55, pp. 2363-2370).

### PREPARATION C :

### 3-[5-(benzyloxy)-1H-indol-3-yl]-1-méthyl-1H-pyrrole-2,5-dione

### Stade A : 3-[5-(behzyloxy)-1H-indol-3-yl]-1-méthyl-2,5-pyrrolidinedione

Un mélange de 5-benzyloxy-indole (8 mmol) et de N-méthylmaléimide (8 mmol) dans 8 ml d'acide acétique sont mis sous reflux pendant 48 heures. L'acide acétique est évaporé. Une purification par chromatographie sur gel de silice (acétate d'éthyle/cyclohexane : 2/8 à 7/3) permet d'obtenir le produit attendu.
Point de fusion : 49-53°C
IR (KBr) : ν_{C=O} = 1690, 1700 cm⁻¹ ; ν_{NH} = 3300-3500 cm⁻¹
Spectre de masse (FAB) : 335,14 [M+H⁺]

### Stade B : 3-[5-(benzyloxy)-1H-indol-3-yl]-1-méthyl-1H-pyrrole-2,5-dione

Une solution de 2,3-dichloro-5,6-dicyano-1,4-benzoquinone (2 mmol) dans 20 ml de dioxane est additionnée lentement à une solution du composé obtenu au stade précédent (2 mmol) dans 20 ml de dioxane. La réaction est agitée pendant une nuit à température ambiante. Après filtration puis évaporation du dioxane, le mélange réactionnel est repris dans de l'isopropanol pour recristallisation. Le produit attendu est obtenu par filtration et lavage à l'isopropanol du précipité formé.
Point de fusion : 176-182°C
IR (KBr) : ν_{C=O} = 1690, 1700 cm⁻¹ ; ν_{NH} = 3300-3440 cm⁻¹
Spectre de masse (FAB) : 333,12 [M+H⁺]

### PREPARATION D :

### 3-(1H-indol-3-yl)-2,5-furanedione

Un mélange du composé de la préparation B (0,884 mmol) et des pastilles d'hydroxyde de sodium (12,5 mmol) dans 100 ml d'eau distillée est mis sous reflux pendant 2 heures. Après refroidissement du mélange réactionnel, de l'acide chlorhydrique concentré est ajouté goutte à goutte jusqu'à formation d'un précipité. Le produit attendu est isolé par filtration du précipité.
Point de fusion : 210-214°C
IR (KBr) : ν_{C=O} = 1740, 1800 cm⁻¹ ; ν_{NH} = 3320 cm⁻¹

### PREPARATION E :

### 3-(1H-pyrrolo[2,3-b]pyridin-3-yl)-1H-pyrrole-2,5-dione

### Stade A : 3-bromo-4-(1H-pyrrolo[2,3-b]pyridin-3-yl)-1H-pyrrole-2,5-dione

Une solution de bromure d'éthylmagnésium est préparée à partir du magnésium (12,7 mmol) en suspension dans le bromoéthane (12,7 mmol) et le tétrahydrofurane sec (5 ml). La solution est agitée 1 heure à température ambiante puis du 7-azaindole (12,7 mmol), dissous dans 40 ml de toluène anhydre, est ajouté goutte à goutte. Après 1 heure 30 d'agitation à température ambiante, une solution de 2,3-dibromomaléimide (3,53 mmol), dans 40 ml de toluène anhydre, est additionnée goutte à goutte. Après 20 minutes, 60 ml de dichlorométhane sec sont ajoutés, puis le mélange réactionnel est laissé sous agitation pendant 75 heures à 40°C puis hydrolysé avec une solution aqueuse saturée de chlorure d'ammonium. Le produit organique est extrait avec de l'acétate d'éthyle, puis les phases organiques rassemblées, séchées sur sulfate de magnésium et filtrées. Après évaporation du solvant, et purification du résidu par chromatographie sur gel de silice (cyclohexane/acétate d'éthyle : 3/2), le produit attendu est isolé.

### Stade B : 3-(1H-pyrrolo[2,3-b]pyridin-3-yl)-2,5-pyrrolidinedione

Un mélange du composé obtenu au stade précédent (0,327 mmol) et d'une quantité catalytique de charbon palladié à 10 % dans le méthanol (40 ml) est hydrogéné à une atmosphère pendant 24 heures. Le mélange est filtré sur célite et le produit attendu est obtenu après purification du résidu par chromatograhie sur gel de silice en utilisant comme éluant de l'acétate d'éthyle.

### Stade C : 3-(1H-pyrrolo[2,3-b]pyridin-3-yl)-1H-pyrrole-2,5-dione

Le produit attendu est obtenu selon le procédé décrit au stade B de la préparation C à partir du composé du stade précédent.

### PREPARATION F :

### 1-méthyl-3-[1-(2,3,4,6-tétra-O-acétyl-β-D-glucopyranosyl)-1H-pyrrolo[2,3-b]pyridin-3-yl]-1H-pyrrole-2,5-dione

### Stade A : 3-bromo-1-méthyl-4-(1H-pyrrolo[2,3-b]pyridin-3-yl)-1H-pyrrole-2,5-dione

Le produit attendu est obtenu selon le procédé décrit au stade A de la préparation E en utilisant du N-méthyl-2,3-dibromomaléimide comme substrat.
Point de fusion : 158°C

### Stade B : 3-bromo-1-méthyl-4-[1-(2,3,4,6-tétra-O-acétyl-β-D-glucopyranosyl)-1H-pyrrolo[2,3-b]pyridin-3-yl]-1H-pyrrole-2,5-dione

A une solution du composé du stade précédent (0,927 mmol) dissous dans 40 ml de tétrahydrofurane sec, sont ajoutés du 2,3,4,6-tétra-O-acétylglucopyranose (1,95 mmol) et de la triphénylphosphine (1,95 mmol) est rajouté goutte à goutte. La température est remontée lentement jusqu'à température ambiante, puis le mélange réactionnel laissé sous agitation encore 15 heures. Après hydrolyse, le produit organique est extrait avec de l'acétate d'éthyle. Les phases organiques sont rassemblées, séchées sur sulfate de magnésium, filtrées et le solvant évaporé. Le produit attendu est obtenu après purification par chromatographie sur gel de silice.

### Stade C : 1-méthyl-3-[1-(2,3,4,6-tétra-O-acétyl-β-D-glucopyranosyl)-1H-pyrrolo [2,3-b]pyridin-3-yl]-2,5-pyrrolidinedione

Le produit attendu est obtenu selon le procédé décrit au stade B de la préparation E à partir du composé du stade précédent.

### Stade D : 1-méthyl-3-[1-(2,3,4,6-tétra-O-acétyl-β-D-glucopyranosyl)-1H-pyrrolo [2,3-b]pyridin-3-yl]-1H-pyrrole-2,5-dione

Le produit attendu est obtenu selon le procédé décrit au stade B de la préparation C à partir du composé du stade précédent.

### PREPARATION G :

### 1-méthyl-3-(1H-pyrrolo[2,3-b]pyridin-3-yl)-1H-pyrrole-2,5-dione

### Stade A : 1-méthyl-3-(1H-pyrrolo[2,3-b]pyridin-3-yl)-2,5-pyrrolidinedione

Un mélange du composé obtenu au stade A de la préparation F (0,65 mmol) et de charbon palladié à 10% (20 mg) dans le méthanol (40 mL) est hydrogéné à 1 atmosphère pendant 3,5 heures. Le mélange est filtré sur célite. Le filtrat est évaporé et le produit attendu est obtenu après purification du résidu par chromatographie sur gel de silice flash (AcOEt puis AcOEt/MeOH 9:1).
Point de fusion : 199-202°C
IR (KBr) : ν_{C=O} = 1690, 1770 cm⁻¹ ; ν_{NH} = 3250-3500 cm⁻¹

### Stade B : 1-méthyl-3-(1H-pyrrolo[2,3-b]pyridin-3-yl)-1H-pyrrole-2,5-dione

Le produit attendu est obtenu selon le procédé décrit au stade B de la préparation C à partir du composé du stade précédent.
Point de fusion : >250°C
IR (KBr) : ν_{C=O} = 1700, 1760 cm ⁻¹ ; ν_{NH} = 3300-3600 cm⁻¹

### PREPARATION H :

### 3-[5-(benzyloxy)-1H-indol-3-yl]-1H-pyrrole-2,5-dione

### Stade A : 3-[5-(benzyloxy)-1H-indol-3-yl]-2,5-pyrrolidinedione

Le produit attendu est obtenu selon le procédé décrit au stade A de la préparation C en remplaçant le N-méthylmaléimide par le maléimide.
Point de fusion : 175°C
IR (KBr) : ν_{C=O} = 1690, 1780 cm⁻¹ ; ν_{NH} = 3210-3320 cm⁻¹

### Stade B : 3-[5-(benzyloxy)-1H-indol-3-yl]-1H-pyrrole-2,5-dione

Le produit attendu est obtenu selon le procédé décrit au stade B de la préparation C à partir du composé du stade précédent.
Point de fusion : 211°C
IR (KBr) : ν_{C=C} = 1600 cm⁻¹ ; ν_{C=O} = 1705, 1755 cm⁻¹ ; ν_{NH} = 3150-3450 cm⁻¹

### PREPARATION I :

### 3-(5-bromo-1H-indol-3-yl)-1H-pyrrole-2,5-dione

### Stade A : 3-(5-bromo-1H-indol-3-yl)-2,5-pyrrolidinedione

Le produit attendu est obtenu selon le procédé décrit au stade A de la préparation C en remplaçant le N-méthylmaléimide par le maléimide et le 5-benzyloxy-indole par le 5-bromo-indole.
Point de fusion : 208-215°C
IR (KBr) : ν_{C=O}= 1700, 1755 cm⁻¹ ; ν_{NH} = 3450 cm⁻¹

### Stade B : 3-(5-bromo-1H-indol-3-yl)-1H-pyrrole-2,5-dione

Le produit attendu est obtenu selon le procédé décrit au stade B de la préparation C à partir du composé du stade précédent.
Point de fusion : 268°C
IR (KBr) : ν_{C=C} = 1595 cm⁻¹ ; ν_{C=O} = 1705, 1750 cm⁻¹ ; ν_{NH} = 3200,3340 cm⁻¹

### PREPARATION J :

### 3-(5-chloro-1H-indol-3-yl)-1H-pyrrole-2,5-dione

### Stade A : 3-(5-chloro-1H-indol-3-yl)-2,5-pyrrolidinedione

Le produit attendu est obtenu selon le procédé décrit au stade A de la préparation I en remplaçant le 5-bromo-indole par le 5-chloro-indole.
IR (KBr) : ν_{C=O} = 1700, 1780 cm⁻¹ ; ν_{NH} = 3200-3500 cm⁻¹

### Stade B : 3-(5-chloro-1H-indol-3-yl)-1H-pyrrole-2,5-dione

Le produit attendu est obtenu selon le procédé décrit au stade B de la préparation C à partir du composé du stade précédent.
Point de fusion : 254-264°C
IR (KBr) : ν_{C=C} = 1605 cm⁻¹ ; ν_{C=O}: = 1710, 1750 cm⁻¹ ; ν_{NH} = 3100-3350 cm⁻¹

### PREPARATION K :

### 3-(5-fluorto-1H-indol-3-yl)-1H-pyrrole-2,5-dione

### Stade A : 3-(5-fluoro-1H-indol-3-yl)-2,5-pyrrolidinedione

Le produit attendu est obtenu selon le procédé décrit au stade A de la préparation I en remplaçant le 5-bromo-indole par le 5-fluoro-indole.
Point de fusion : 190-195°C
IR (KBr) : ν_{C=C} = 1690,1775 cm⁻¹ ; ν_{NH} = 3360 cm⁻¹

### Stade B : 3-(5-fluoro-1H-indol-3-yl)-1H-pyrrole-2,5-dione

Le produit attendu est obtenu selon le procédé décrit au stade B de la préparation C à partir du composé du stade précédent.
Point de fusion : 255-265°C
IR (KBr) : ν_{C=C} = 1605 cm⁻¹ ; ν_{C=O} = 1720, 1750 cm⁻¹ ; ν_{NH} = 3150 - 3350 cm⁻¹

### PREPARATION L :

### 3-(5-hydroxy-1H-indol-3-yl)-1H-pyrrole-2,5-dione

### Stade A : 3-(5-hydroxy-1H-indol-3-yl)-2,5-pyrrolidinedione

A une solution du composé du stade A de la préparation H (450 mg) dans le méthanol sec (90 mL) est ajouté du charbon palladié à 10% (135 mg). Après avoir purgé sous vide pendant 20 minutes, le milieu réactionnel est placé sous atmosphère d'H₂ (1 atm) pendant 3 heures. Après filtration sur célite et évaporation du filtrat, le produit attendu est obtenu.
IR (film) : ν_{C=C} = 1700 cm⁻¹ ; ν_{NH,OH} = 3000-3700 cm⁻¹

### Stade B : 3-(5-hydroxy-1H-indol-3-yl)-1H-pyrrole-2,5-dione

Le produit attendu est obtenu selon le procédé décrit au stade B de la préparation C à partir du composé du stade précédent.
Point de fusion : 292-298°C
IR (KBr) : ν_{C=C} =1610 cm⁻¹ ; ν_{C=O} = 1690, 1760 cm⁻¹ ; ν_{NH.OH} = 3260, 3370, 3430 cm⁻¹

### PREPARATION M :

### 3-[1-(2,3,4,6-tétra-O-benzyl-β-D-glucopyranosyl)-1H-indol-3-yl]-1H-pyrrole-2,5-dione

### Stade A : 3-bromo-4-(1H-indol-3-yl)-1-méthyl-1H-pyrrole-2,5-dione

Une solution contenant 1,445 g d'indole dissous dans 29 ml de tétrahydrofurane sec est portée entre -20 et -10°C sous argon, puis 26 ml de LiHMDS (1 M dans l'hexane) sont ajoutés goutte à goutte en 15 minutes. Après 45 minutes à -10°C, la solution est diluée avec 15 ml de tétrahydrofurane supplémentaire et une solution contenant 2 g de N-méthyl-2,3-dibromomaleimide dissous dans 17 ml de tétrahydrofurane est ajoutée goutte à goutte en 30 minutes. Après 15 minutes à -10°C et 15 minutes à 0°C, la réaction est stoppée par l'ajout à 0°C de 50 ml d'une solution d'acide chlorhydrique 0,3 N. Le mélange réactionnel est extrait à l'acétate d'éthyle, les phases organiques lavées avec une solution saturée en NaCl, séchées sur MgSO₄ puis évaporées sous pression réduite. Le produit souhaité est précipité avec du méthanol.
Point de fusion = 167-168°C.

### Stade B : 3-bromo-1-méthyl-4-[1-(2,3,4,6-tétra-O-benzyl-β-D-glucopyranosyl)-1H-indol-3-yl]-1H-pyrrole-2,5-dione

Une solution du composé du stade A précédent, de PPh₃ (3 équiv.) et de 2,3,4,6-tétra-*O*-benzyl-D-glucopyranosyl (3 équiv.) dans le THF sec est refroidie à -78°C. Le DIAD (3 équiv.) est alors additionné goutte à goutte. Le milieu réactionnel est agité pendant 4 heures, en laissant réchauffer à température ambiante. Une solution d'HC1 0,2 M est versée et le mélange est extrait avec de l'acétate d'éthyle. La phase organique est lavée successivement avec une solution aqueuse saturée en NaHCO₃ et à l'eau puis séchée sur MgSO₄. Après filtration et évaporation du solvant et chromatographie sur gel de silice (toluène/acétate d'éthyle : 50/1), le produit attendu est obtenu.
Point de fusion : 55°C
IR (KBr) : ν_{C=O} = 1640 cm ⁻¹ ; ν_{C=O} = 1710, 1770 cm⁻¹ ; ν_{NH.OH} = 3200-3600 cm⁻¹

### Stade C : 1-méthyl-3-[1-(2,3,4,6-tétra-O-benzyl-β-D-glucopyranosyl)-1H-indol-3-yl]-2,5-pyrrolidinedione

Une solution du composé du stade B précédent dans du méthanol et du THF sec est hydrogénée (1 atm) pendant 3 heures en présence de charbon palladié à 10% et de pyridine (0,5 équiv.). Après filtration sur célite et évaporation du filtrat, et chromatographie sur gel de silice (cyclohexane/acétate d'éthyle) le produit attendu est obtenu.

### Stade D : 1-méthyl-3-[1-(2,3,4,6-tétra-O-benzyl-β-D-glucopyranosyl)-1H-indol-3-yl]-1H-pyrrole-2,5-dione

Le produit attendu est obtenu selon le procédé décrit au stade B de la préparation C à partir du composé du stade précédent.

### Stade E: 3-[1-(2,3,4,6-tétra-O-benzyl-β-D-glucopyranosyl)-1H-indol-3-yl]-1H-pyrrole-2,5-dione

Un mélange du composé du stade D précédent et de NaOH dans l'eau distillée est chauffée à reflux pendant 2 heures. Après refroidissement, de l'acide chlorhydrique concentré est versé goutte à goutte jusqu'à formation d'un précipité jaune. Le solide jaune est filtré sur fritté et lavé avec de l'eau donnant l'anhydride désiré. Celui-ci est ensuite traité par une solution ammoniac dans le THF pour conduire au composé attendu.

### PREPARATION N :

### 3-{[1-(2,3,4,6-tétra-O-benzyl-β-D-glucopyranosyl)]-1H-pyrrolo[2,3-b]pyridin-3-yl}-1H-pyrrole-2,5-dione

### Stade A : 3-bromo-1-méthyl-4-[1-(2,3,4,6-tétra-O-benzyl-β-D-glucopyranosyl)-1H-pyrrolo[2,3-b]pyridin-3-yl]-1H-pyrrole-2,5-dione

A une solution du composé du stade A de la préparation G (50 mg) dissous dans 4 mL de THF sec, sont ajoutés du 2,3,4,6-*O*-benzylglucopyranosyl (264 mg) et de la triphénylphosphine (128 mg). Le mélange réactionnel est refroidi à -78 °C, puis le DIAD (97 µL) est ajouté goutte à goutte. La température est remontée lentement jusqu'à température ambiante, puis le mélange réactionnel est laissé sous agitation encore 15 heures. Après hydrolyse (40 mL d'eau), le produit organique est extrait avec de l'acétate d'éthyle (3 x 150 mL). Les phases organiques sont rassemblées, séchées sur sulfate de magnésium, filtrées et le solvant évaporé. Une purification par chromatographie sur gel de silice (cyclohexane/acétate d'éthyle : 8/2 à 7/3) permet d'isoler le produit attendu .
IR (KBr) : ν_{C=O} = 1710, 1740, 1760 cm⁻¹

### Stade B : 1-méthyl-3-[1-(2,3,4,6-tétra-O-benzyl-β-D-glucopyranosyl)-1H-pyrrolo[2,3-b]pyridin-3-yl]-2,5-pyrrolidinedione

A une suspension de composé du stade A précédent (65 mg) dans 10 mL d'acétate d'éthyle sont ajoutés NaHCO₃ (66 mg) et du Pd/C 10 % (65 mg). Le mélange est placé sous atmosphère d'hydrogène (1 bar) à température ambiante pendant 24h. Une filtration sur célite permet d'éliminer le catalyseur et le filtrat est évaporé sous pression réduite. Une purification par chromatograhie sur gel de silice flash (cyclohexane/acétate d'éthyle : 8/2 à 7/3) permet d'isoler le produit attendu.
IR (KBr) : ν_{C=O} = 1710-1750 cm⁻¹

### Stade C : 1-méthyl-3-{[1-(2,3,4,6-tétra-O-benzyl-β-D-glucopyranosyl)]-1H-pyrrolo [2,3-b]pyridin-3-yl}-1H-pyrrole-2,5-dione

Le produit attendu est obtenu selon le procédé décrit au stade B de la préparation C à partir du composé du stade précédent.

### Stade D : 3-{[1-(2,3,4,6-tétra-O-benzyl-β-D-glucopyranosyl)]-1H-pyrrolo[2,3-pyridin-3-yl}-2,5-furanedione

A une suspension du composé du stade C précédent (1 mmol) dans l'eau sont ajoutés NaOH en pastilles (14 mmol) et du THF. Le mélange est laissé sous agitation à température ambiante pendant 2 heures, puis acidifié à pH 1 par addition d'acide chlorydrique concentré. Après agitation 30 minutes le produit organique est extrait avec de l'acétate d'éthyle (3 x 150 mL). Les phases organiques sont rassemblées, séchées sur sulfate de magnésium, filtrées et le solvant évaporé. Une purification par chromatographie sur gel de silice permet d'isoler le produit attendu.

### Stade E : 3-{[1-(2,3,4,6-tétra-O-benzyl-β-D-glucopyranosyl)]-1H-pyrrolo[2,3-b] pyridin-3-yl}-1H-pyrrole-2,5-dione

Dans un tube scellé, une solution du composé du stade D précédent dans THF est ajoutée à une solution de THF saturé en NH₃. Le mélange réactionnel est laissé sous agitation à 80 °C pendant 18 heures. Après refroidissement, le mélange est versé dans l'eau et extrait à l'acétate d'éthyle plusieurs fois. Les phases organiques sont rassemblées, séchées sur sulfate de magnésium, filtrées et le solvant évaporé. Une purification par chromatographie sur gel de silice permet d'isoler le produit attendu.

Les composés des préparations O à AP sont obtenus selon le procédé de la préparation C à partir des indoles correspondants et en remplaçant le N-méthylmaléimide par le maléimide.

### PREPARATION O :

### 3-(5-amino-1H-indol-3-yl)-1H-pyrrole-2,5-dione

### PREPARATION P :

### 3-(4-amino-1H-indol-3-yl)-1H-pyrrole-2,5-dione

### PREPARATION Q :

### 3-(5,6-diméthoxy-1H-indol-3-yl)-1H-pyrrole-2,5-dione

### PREPARATION R :

### 3-(4-nitro-1H-indol-3-yl)-1H-pyrrole-2,5-dione

### PREPARATION S :

### 3-(4-fluoro-1H-indol-3-yl)-1H-pyrrole-2,5-dione

### PREPARATION T :

### 3-(6-fluoro-1H-indol-3-yl)-1H-pyrrole-2,5-dione

### PREPARATION U :

### 3-(4-hydroxy-1H-indol-3-yl)-1H-pyrrole-2,5-dione

### PREPARATION V :

### 3-(5-hydroxy-1H-indol-3-yl)-1H-pyrrole-2,5-dione

### PREPARATION W :

### 3-(4-méthoxy-1H-indol-3-yl)-1H-pyrrole-2,5-dione

### PREPARATION X :

### 3-(5-méthoxy-1H-indol-3-yl)-1H-pyrrole-2,5-dione

### PREPARATION Y :

### 3-(6-méthoxy-1H-indol-3-yl)-1H-pyrrole-2,5-dione

### PREPARATION Z :

### 3-(7-méthoxy-1H-indol-3-yl)-1H-pyrrole-2,5-dione

### PREPARATION AA :

### 3-(4-méthyl-1H-indol-3-yl)-1H-pyrrole-2,5-dione

### PREPARATION AB :

### 3-(5-méthyl-1H-indol-3-yl)-1H-pyrrole-2,5-dione

### PREPARATION AC :

### 3-(6-méthyl-1H-indol-3-yl)-1H-pyrrole-2,5-dione

### PREPARATION AD :

### 3-(7-méthyl-1H-indol-3-yl)-1H-pyrrole-2,5-dione

### PREPARATION AE :

### 3-(4-chloro-1H-indol-3-yl)-1H-pyrrole-2,5-dione

### PREPARATION AF :

### 3-(5-chloro-1H-indol-3-yl)-1H-pyrrole-2,5-dione

### PREPARATION AG :

### 3-(6-chloro-1H-indol-3-yl)-1H-pyrrole-2,5-dione

### PREPARATION AH :

### 3-(7-chloro-1H-indol-3-yl)-1H-pyrrole-2,5-dione

### PREPARATION AI :

### 3-(4-bromo-1H-indol-3-yl)-1H-pyrrole-2,5-dione

### PREPARATION AJ :

### 3-(6-bromo-1H-indol-3-yl)-1H-pyrrole-2,5-dione

### PREPARATION AK :

### 3-(7-bromo-1H-indol-3-yl)-1H-pyrrole-2,5-dione

### PREPARATION AL :

### 3-(5-méthoxy-4-méthyl-1H-indol-3-yl)-1H-pyrrole-2,5-dione

### PREPARATION AM :

### 3-[(7-(benzyloxy)-1H-indol-3-yl]-1H-pyrrole-2,5-dione

### PREPARATION AN :

### 3-[6-(benzyloxy)-1H-indol-3-yl]-1H-pyrrole-2,5-dione

### PREPARATION AO :

### 3-[5-(benzyloxy)-6-méthoxy-1H-indol-3-yl]-1H-pyrrole-2,5-dione

### PREPARATION AP :

### 3-(1-méthyl-1H-indol-3-yl]-1H-pyrrole-2,5-dione.

### EXEMPLE 1 : 1H-dipyrrolo[3,4-a:3,4-c]carbazole-1,3,4,6(2H,5H,7H)-tétrone

Le composé de la préparation A (0,388 mmol) est chauffé sous reflux dans 24 ml de dioxane en présence d'acide trifluoroacétique (400 µl) pendant 24 heures. Après évaporation du solvant, les cristaux sont repris avec de l'acétate d'éthyle et lavés avec une solution saturée d'hydrogénocarbonate de sodium et une solution saturée de chlorure de sodium. Le produit attendu est obtenu par filtration sur fritté des cristaux.
Point de fusion : > 300°C
IR (KBr) : ν_{C=O} = 1690, 1730, 1745, 1780 cm⁻¹ ; ν_{NH} = 3280-3380 cm⁻¹
Spectrométrie de masse (FAB) : 306,05 [M+H⁺]

### EXEMPLE 2 : 2,5-diméthyl-1H-dipyrrolo[3,4-a:3,4-c]carbazole-1,3,4,6(2H,5H,7H)-tétrone

Un mélange du composé de la préparation B (1 mmol) et de N-méthylmaléimide (1,10 mmol) dans 17 ml de para-xylène est mis sous reflux pendant 24 heures. Après refroidissement, le précipité jaune est filtré puis lavé au para-xylène. Une chromatographie sur colonne de silice (acétate d'éthyle/cyclohexane : 1/1 ; acétate d'éthyle ; acétate d'éthyle/méthanol : 98/2) permet d'obtenir un mélange d'isomères qui est chauffé sous reflux dans 25 ml de dioxane en présence d'acide trifluoroacétique pendant 84 heures. Après évaporation du solvant, les cristaux sont repris avec de l'acétate d'éthyle et lavés avec une solution saturée d'hydrogénocarbonate de sodium et une solution saturée de chlorure de sodium. Le produit attendu est obtenu par filtration sur fritté des cristaux.
Point de fusion : > 300°C
IR (KBr) : ν_{C=O} = 1695, 1720 cm⁻¹ ; ν_{NH} = 3410 cm⁻¹
Spectrométrie de masse (FAB) : 334,08 [M+H⁺]

### EXEMPLE 3 : 2-méthyl-1H-dipyrrolo[3,4-a:3,4-c]carbazole-1,3,4,6(2H-5H-7H)-tétrone

Le produit attendu est obtenu selon le procédé décrit dans l'exemple 2 à partir du composé de la préparation B et de maléimide.
Point de fusion : > 300°C
IR (KBr) : ν_{C=O} = 1710, 1720, 1760, 1780 cm⁻¹ ; ν_{NH} = 3260-3395 cm⁻¹
Spectrométrie de masse (FAB) : 320,06 [M+H⁺]

### EXEMPLE 4 : 10-(benzyloxy)-2,5-diméthyl-1H-dipyrrolo[3,4-a:3,4-c]carbazole-1,3,4,6(2H,5H,7H)-tétrone

Le produit attendu est obtenu selon le procédé décrit dans l'exemple 2 à partir du composé de la préparation C et de N-méthylmaléimide.
Point de fusion : > 300°C
IR (KBr) : ν_{C=O} = 1700, 1720, 1775 cm⁻¹ ; ν_{NH} = 3480 cm⁻¹
Spectrométrie de masse (FAB) : 440,12 [M+H⁺]

### EXEMPLE 5 : 5-méthylfuro[3,4-c]pyrrolo[3,4-a]carbazole-1,3,4,6(2H,5H,7H)-tétrone

Le produit attendu est obtenu selon le procédé décrit dans l'exemple 2 à partir du composé de la préparation D et de N-méthylmaléimide.
Point de fusion : 294°C (décomposition)
IR (KBr) : ν_{C=O} = 1775, 1840 cm⁻¹ ; ν_{NH} = 3370 cm⁻¹
Spectrométrie de masse (FAB) : 321,05 [M+H⁺]

### EXEMPLE 6 : Chlorhydrate de 2-[2-(diéthylamino)éthyl]-5-méthyl-1H-dipyrrolo [3,4-a:3,4-c]carbazole-1,3,4,6(2H,5H,7H)-tétrone

A une solution du composé de l'exemple 5 (0,088 mmol) dissous dans 5,2 ml de tétrahydrofurane anhydre est ajoutée goutte à goutte la *N*-*N*-diéthyléthylènediamine (0,132 mmol). Le mélange est porté à 65°C pendant 4 jours à l'abri de la lumière puis refroidi et repris avec un mélange d'une solution aqueuse d'acide chlorhydrique 1N (40 ml) et d'acétate d'éthyle. Le produit organique est extrait avec de l'acétate d'éthyle. La phase aqueuse est reprise avec de l'acétate d'éthyle et le pH est ajusté à 12 par addition d'une solution aqueuse saturée de bicarbonate de sodium. Le produit organique est extrait avec de l'acétate d'éthyle. Les phases organiques sont rassemblées, séchées sur sulfate de magnésium, filtrées et le solvant est évaporé à froid. A une solution refroidie à 0°C de l'amine ainsi obtenue, dissous dans 400 µl de méthanol, est ajoutée goutte à goutte une solution aqueuse d'acide chlorhydrique 1N (190 µl). Le mélange est agité pendant 30 minutes. Le solvant est évaporé permettant d'isoler le produit attendu.
Point de fusion : 184°C (décomposition)
IR (KBr) : ν_{C=O} = 1710, 1720, 1765, 1775 cm⁻¹ ; ν_{NH} = 3300-3600 cm⁻¹
Spectrométrie de masse (FAB) : 419,17 [M+H⁺]

### EXEMPLE 7 : 1H-pyrido[2,3-b]dipyrrolo[3,4-e:3,4-g]indole-1,3,4,6(2H,5H,7H)-tétrone

Le produit attendu est obtenu selon le procédé décrit dans l'exemple 2 à partir du composé de la préparation E et de maléimide.

### EXEMPLE 8 : 2-méthyl-7-(2,3,4,6-tétra-O-acétyl-β-D-glucopyranosyl)-1H-pyrido [2,3-b]dipyrrolo[3,4-e:3,4-g]indole-1,3,4,6(2H,5H,7H)-tétrone

Le produit attendu est obtenu selon le procédé décrit dans l'exemple 2 à partir du composé de la préparation F et de maléimide.

### EXEMPLE 9 : 2-méthyl-1H-pyrido[2,3-b]dipyrrolo[3,4-e:3,4-g]indole-1,3,4,6(2H,5H,7H)-tétrone

Un mélange du composé de la préparation G (55,0 mg) et de maléimide (25,9 mg) dans le xylène (5 ml) est chauffé à reflux pendant 20 heures. Après refroidissement, le mélange est filtré et lavé au xylène. Le solide obtenu (70,8 mg) est chauffé à reflux pendant 3 jours dans le dioxane (5 ml) en présence de 2,3-dichloro-5,6-dicyano-1,4-benzoquinone (115,5 mg). Après évaporation du solvant, le résidu est repris à l'eau et le solide obtenu est filtré et lavé à l'eau et à l'acétate d'éthyle, ce qui permet d'obtenir le produit attendu.
Point de fusion : >300°C
IR (KBr) : ν_{C=C} = 1600 cm⁻¹ ; ν_{C=O} = 1710, 1730, 1770, 1780 cm⁻¹ ; ν_{NH} = 3200 cm⁻¹

### EXEMPLE 10 : 2-méthylfuro[3,4-a]pyrrolo[3,4-c]carbazole-1,3,4,6(2H,7H)-tétrone

Un mélange du composé de la préparation B (315 mg) et d'anhydride maléique (164 mg) dans le *p*-xylène (24 mL) est porté à reflux pendant 40 heures. Après retour à température ambiante, le mélange est filtré et les cristaux sont lavés au *p*-xylène puis séchés. Le solide obtenu (357 mg) est chauffé à reflux dans le dioxane (8 mL) pendant 3 jours en présence de 2,3-dichloro-5,6-dicyano-1,4-benzoquinone (540 mg). Après retour à température ambiante, de l'eau et de l'acétate d'éthyle sont ajoutés. Le solide à l'interface est filtré sur fritté, lavé à l'eau et à l'acétate d'éthyle, ce qui permet d'isoler le produit attendu.
Point de fusion : >300°C
IR (KBr) : ν_{C=O} = 1705, 1760, 1835 cm⁻¹; ν_{NH} = 3770 cm⁻¹

### EXEMPLE 11 : Chlorhydrate de 5-[2-(diéthylamino)éthyl]-2-méthyl-1H-dipyrrolo[3,4-a:3,4-c]carbazole-1,3,4,6(2H,5H,7H)-tétrone

A une solution du composé de l'exemple 10 dans le tétrahydrofurane sec (5,5 mL) est ajoutée goutte à goutte la N-N-diéthyléthylènediamine (20 µl). Le mélange est porté à 65 °C pendant 4 jours à l'abri de la lumière. Après retour à température ambiante le milieu est évaporé à sec, repris dans 1 mL d'anhydride acétique en présence d'AcONa (75 mg) puis chauffé à 90°C pendant 4 heures. Le brut réactionnel est refroidi puis repris avec un mélange d'une solution aqueuse d'acide chlorhydrique 1N (40 mL) et d'acétate d'éthyle. La phase aqueuse est alors reprise avec de l'acétate d'éthyle et traitée par une solution aqueuse saturée de bicarbonate de sodium. Le produit organique est alors extrait avec de l'acétate d'éthyle (3 x 50 mL). Les phases organiques sont rassemblées, séchées sur sulfate de magnésium et le solvant est évaporé à froid. L'amine libre ainsi obtenue (32,8 mg) est reprise dans le méthanol (1mL), puis refroidie à 0°C. Une solution aqueuse d'acide chlorhydrique 1N (172 µL) est alors ajoutée goutte à goutte. Le mélange est agité pendant 30 minutes. Le solvant est évaporé ce qui permet d'obtenir le produit attendu.
Point de fusion : =278-280°C
IR (KBr) : ν_{C=O} = 1710, 1770 cm⁻¹ ; ν_{NH} = 3200, 3600 cm⁻¹

### EXEMPLE 12 : 5-méthyl-1H-dipyrrolo[3,4-a:3,4-c]carbazole-1,3,4,6(2H,5H,7H)-tétrone

Le produit attendu est obtenu selon le procédé décrit dans l'exemple 9 à partir du composé de la préparation A et en remplaçant le maléimide par le N-méthylmaléimide.
Point de fusion : >300°C
IR (KBr) : ν_{C=O} = 1695, 1725, 1765, 1775 cm⁻¹ ; ν_{NH} = 3220, 3330 cm⁻¹

### EXEMPLE 13 : Furo[3,4-a]pyrrolo[3,4-c]carbazole-1,3,4,6(2H,7H)-tétrone

Le produit attendu est obtenu selon le procédé décrit dans l'exemple 10 à partir du composé de la préparation A.
Point de fusion : >300°C
IR (KBr) : ν_{C=C} = 1610 cm⁻¹ ; ν_{C=O} = 1700-1850 cm⁻¹ ; ν_{NH} = 3240, 3380 cm⁻¹

### EXEMPLE 14 : 10-(benzyloxy)-1H-dipyrrolo[3,4-a:3,4-c]carbazole-1,3,4,6(2H,5H,7H)-tétrone

Le produit attendu est obtenu selon le procédé décrit dans l'exemple 9 à partir du composé de la préparation H.
Point de fusion : >300°C
IR (KBr) : ν_{C=O} = 1725, 1755, 1780 cm⁻¹ ; ν_{NH} = 3150-3500 cm⁻¹

### EXEMPLE 15 : 10-bromo-1H-dipyrrolo[3,4-a:3,4-c]carbazole-1,3,4,6(2H,5H,7H)-tétrone

Le produit attendu est obtenu selon le procédé décrit dans l'exemple 9 à partir du composé de la préparation I.
Point de fusion : >300°C
IR (KBr) : ν_{C-C} = 1600 cm⁻¹ ; ν_{C-O} = 1710, 1720, 1760 cm⁻¹ ; ν_{NH} = 3150-3350 cm⁻¹

### EXEMPLE 16 : 10-chloro-1H-dipyrrolo[3,4-a:3,4-c]carbazole-1,3,4,6(2H,5H,7H)-tétrone

Le produit attendu est obtenu selon le procédé décrit dans l'exemple 9 à partir du composé de la préparation J.
Point de fusion : >300°C
IR (KBr) : ν_{C=C} = 1600 cm⁻¹ ; ν_{C-O} = 1710, 1720, 1760 cm⁻¹ ; ν_{NH} = 3120-3380 cm⁻¹

### EXEMPLE 17 : 10-fluoro-1H-dipyrrolo[3,4-a:3,4-c]carbazole-1,3,4,6(2H,5H,7H)-tétrone

Le produit attendu est obtenu selon le procédé décrit dans l'exemple 9 à partir du composé de la préparation K.
Point de fusion : >300°C
IR (KBr) : ν_{C=O} = 1710, 1780 cm⁻¹ ; ν_{NH} = 3100-3350 cm⁻¹

### EXEMPLE 18 : 10-hydroxy-1H-dipyrrolo[3,4-a:3,4-c]carbazole-1,3,4,6(2H,5H,7H) -tétrone

Le produit attendu est obtenu selon le procédé décrit dans l'exemple 9 à partir du composé de la préparation L.
Point de fusion : >300°C
IR (KBr) : ν_{C=O} = 1725, 1770 cm⁻¹ ; ν_{NH} = 3100-3650 cm⁻¹

### EXEMPLE 19 : 7-[1-(2,3,4,6-tétra-O-benzyl-β-D-glucopyranosyl)]-1H-dipyrrolo[3,4-a:3,4-c]carbazole-1,3,4,6(2H,5H,7H)-tétrone

Le produit attendu est obtenu selon le procédé décrit dans l'exemple 2 à partir du composé de la préparation M et de maléimide.

### EXEMPLE 20 : 7-(β-D-glucopyranosyl)-1H-dipyrrolo[3,4-a:3,4-c]carbazole-1,3,4,6(2H,5H,7H)-tétrone

La débenzylation par BBr₃ du composé de l'exemple 19 dans le dichlorométhane permet d'obtenir le composé attendu.

### EXEMPLE 21: 7-[1-(2,3,4,6-tétra-O-benzyl-β-D-glucopyranosyl)]-1H-pyrido[2,3-b] dipyrrolo[3,4-e:3,4-g]carbazole-1,3,4,6(2H,5H,7H)-tétrone

Le produit attendu est obtenu selon le procédé décrit dans l'exemple 9 à partir du composé de la préparation N.

### EXEMPLE 22 : 7-(β-D-glucopyranosyl)]-1H-pyrido[2,3-b]dipyrrolo[3,4-e:3,4-g] indole-1,3,4,6(2H,5H,7H)-tétrone

A une solution du composé de l'exemple 21 dans le dichlorométhane à -78 °C est ajoutée une solution de BBr₃ 1M dans CH₂Cl₂ (8 éq.). Après 10 mn. d'agitation à -78 °C, le mélange réactionnel est hydrolysé et ramené à température ambiante, puis extrait plusieurs fois à l'acétate d'éthyle. La phase organique est séchée sur sulfate de magnésium, puis concentrée sous pression réduite. Une chromatographie sur gel de silice permet d'isoler le produit attendu.

Les produits des exemples 23 à 50 sont obtenus selon le procédé décrit dans l'exemple 9 à partir des composés des préparations O à AP respectivement.

### EXEMPLE 23 : 10-amino-1H-dipyrrolo[3,4-a:3,4-c]carbazole-1,3,4,6(2H,5H,7H)-tétrone

### EXEMPLE 24 : 11-amino-1H-dipyrrolo[3,4-a:3,4-c]carbazole-1,3,4,6(2H,5H,7H)-tétrone

### EXEMPLE 25 : 9,10-diméthoxy-1H-dipyrrolo[3,4-a:3,4-c]carbazole-1,3,4,6-(2H,5H,7H)-tétrone

### EXEMPLE 26 : 11-nitro-1H-dipyrrolo[3,4-a:3,4-c]carbazole-1,3,4,6(2H,5H,7H)-tétrone

### EXEMPLE 27 : 11-fluoro-1H-dipyrrolo[3,4-a:3,4-c]carbazole-1,3,4,6(2H,5H,7H)-tétrone

### EXEMPLE 28 : 9-fluoro-1H-dipyrrolo[3,4-a:3,4-c]carbazole-1,3,4,6(2H,5H,7H)-tétrone

### EXEMPTE 29 : 11-hydroxy-1H-dipyrrolo[3,4-a:3,4-c]carbazole-1,3,4,6(2H,5H,7H)-tétrone

### EXEMPLE 30 : 10-hydroxy-1H-dipyrrolo[3,4-a:3,4-c]carbazole-1,3,4,6(2H,5H,7H)-tétrone

### EXEMPLE 31 : 11-méthoxy-1H-dipyrrolo[3,4-a:3,4-c]carbazole-1,3,4,6(2H,5H,7H)-tétrone

### EXEMPLE 32 : 10-méthoxy-1H-dipyrrolo[3,4-a:3,4-c]carbazole-1,3,4,6(2H,5H,7H)-tétrone

### EXEMPLE 33 : 9-méthoxy-1H-dipyrrolo[3,4-a:3,4-c]carbazole-1,3,4,6(2H,5H,7H)-tétrone

### EXEMPLE 34 : 8-méthoxy-1H-dipyrrolo[3,4-a:3,4-c]carbazole-1,3,4,6(2H,5H,7H)-tétrone

### EXEMPLE 35 : 11-méthyl-1H-dipyrrolo[3,4-a:3,4-c]carbazole-1,3,4,6(2H,5H,7H)-tétrone

### EXEMPLE 36 : 10-méthyl-1H-dipyrrolo[3,4-a:3,4-c]carbazole-1,3,4,6(2H,5H,7H)-tétrone

### EXEMPLE 37 : 9-métbyl-1H-dipyrrolo[3,4-a:3,4-c]carbazole-1,3,4,6(2H,5H,7H)-tétrone

### EXEMPLE 38 : 8-méthyl-1H-dipyrrolo[3,4-a:3,4-c]carbazole-1,3,4,6(2H,5H,7H)-tétrone

### EXEMPLE 39 : 11-chloro-1H-dipyrrolo[3,4-a:3,4-c]carbazole-1,3,4,6(2H,5H,7H)-tétrone

### EXEMPLE 40 : 10-chloro-1H-dipyrrolo[3,4-a:3,4-c]carbazole-1,3,4,6(2H,5H,7H)-tétrone

### EXEMPLE 41 : 9-chloro-1H-dipyrrolo[3,4-a:3,4-c]carbazole-1,3,4,6(2H,5H,7H)-tétrone

### EXEMPLE 42 : 8-chloro-1H-dipyrrolo[3,4-a:3,4-c]carbazole-1,3,4,6(2H,5H,7H)-tétrone

### EXEMPLE 43 : 11-bromo-1H-dipyrrolo[3,4-a:3,4-c]carbazole-1,3,4,6(2H,5H,7H)-tétrone

### EXEMPLE 44 : 9-bromo-1H-dipyrrolo[3,4-a:3,4-c]carbazole-1,3,4,6(2H,5H,7H)-tétrone

### EXEMPLE 45 : 8-bromo-1H-dipyrrolo[3,4-a:3,4-c]carbazole-1,3,4,6(2H,5H,7H)-tétrone

### EXEMPLE 46 : 10-méthoxy-11-méthyl-1H-dipyrrolo[3,4-a:3,4-c]carbazole-1,3,4,6-(2H,5H,7H)-tétrone

### EXEMPLE 47 : 8-(benzyloxy)-1H-dipyrrolo[3,4-a:3,4-c]carbazole-1,3,4,6(2H,5H,7H)-tétrone

### EXEMPLE 48 : 9-(benzyloxy)-1H-dipyrrolo[3,4-a:3,4-c]carbazole-1,3,4,6(2H,5H,7H)-tétrone

### EXEMPLE 49 : 10-(benzyloxy)-9-méthoxy-1H-dipyrrolo[3,4-a:3,4-c]carbazole-1,3,4,6(2H,5H,7H)-tétrone

### EXEMPLE 50 : 7-méthyl-1H-dipyrrolo[3,4-a:3,4-c]carbazole-1,3,4,6(2H,5H,7H)-tétrone

### EXEMPLE 51 : 2-hydroxy-5-méthyl-1H-dipyrrolo[3,4-a:3,4-c]carbazole-1,3,4,6-(2H,5H,7H)-tétrone

Le produit attendu est obtenu selon le procédé décrit dans l'exemple 6 en remplaçant la N,N-diéthyléthylènediamine par l'hydroxylamine.

### EXEMPLE 52 : 2-amino-5-méthyl-1H-dipyrrolo[3,4-a:3,4-c]carbazole-1,3,4,6-(2H,5H,7H)-tétrone

Le produit attendu est obtenu selon le procédé décrit dans l'exemple 6 en remplaçant la N,N-diéthyléthylènediamine par l'hydrazine.

### EXEMPLE 53 : 2-(diméthylamino)-5-méthyl-1H-dipyrrolo[3,4-a:3,4-c]carbazole-1,3,4,6-(2H,5H,7H)-tétrone

Le produit attendu est obtenu selon le procédé décrit dans l'exemple 6 en remplaçant la N,N-diéthyléthylènediamine par la 1,1-diméthylhydrazine.

### EXEMPLE 54 : 2-(3-hydroxypropyl)-5-méthyl-1H-dipyrrolo[3,4-a:3,4-c]carbazole-1,3,4,6(2H,5H,7H)-tétrone

Le produit attendu est obtenu selon le procédé décrit dans l'exemple 6 en remplaçant la N,N-diéthyléthylènediamine par le 3-amino-1-propanol.

### EXEMPLE 55 : 2-(3-méthoxypropyl)-5-métbyl-1H-dipyrrolo[3,4-a:3,4-c] carbazole-1,3,4,6(2H,5H,7H)-tétrone

Le produit attendu est obtenu selon le procédé décrit dans l'exemple 6 en remplaçant la N,N-diéthyléthylènediamine par la 3-méthoxypropylamine.

### ETUDE PHARMACOLOGIQUE DES COMPOSES DE L'INVENTION

### EXEMPLE 56 Activité in vitro

Trois lignées cellulaires ont été utilisées :
- ***Leucémie murine L1210***
- ***Carcinome pulmonaire humain non à petites cellues A549***
- ***Carcinome de la prostate DU145***

La leucémie murine L1210 a été utilisée in vitro. Les cellules sont cultivées dans du milieu de culture RPMI 1640 complet contenant 10 % de sérum de veau foetal, 2 mM de glutamine, 50 U/ml de pénicilline, 50 µg/ml de streptomycine et 10 mM d'Hepes, pH : 7,4. Les cellules sont réparties dans des microplaques et exposées aux composés cytotoxiques pendant 4 temps de doublement, soit 48 heures. Le nombre de cellules viables est ensuite quantifié par un essai colorimétrique, le Microculture Tetrazolium Assay (J. Carmichael et al., Cancer Res.; 47, 936-942, (1987)). Les résultats sont exprimés en IC₅₀, concentration encytotoxique qui inhibe à 50 % la prolifération des cellules traitées. A titre d'exemple, le composé de l'exemple 2 présente des IC₅₀ de 6,8 µM sur L1210, 4,7 µM sur A549 et 5,4 µM sur DU 145.

### EXEMPLE 57 : Composition pharmaceutique : soluté injectable

Composé de l'exemple 1 10 mg
Eau distillée pour préparations injectables 25 ml

## Revendications

1. Composés de formule (I) : 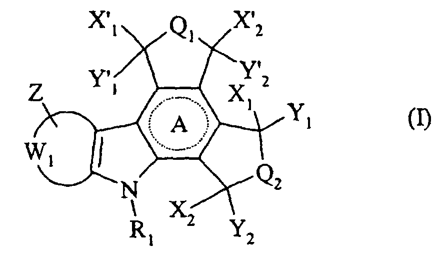 dans laquelle :
• **A** représente un cycle saturé, partiellement ou totalement insaturé pouvant éventuellement conférer un caractère aromatique au cycle,
• W₁ représente, avec les atomes de carbone auxquels il est lié, un groupement phényle ou un groupement pyridinyle,
• **Z** représente un ou plusieurs groupements identiques ou différentes de formule U-V dans laquelle :
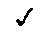 U représente une liaison simple, une chaîne alkylène (C₁-C₆) linéaire ou ramifiée ou une chaîne alkényle (C₂-C₆) linéaire ou ramifiée, éventuellement substituée par un ou plusieurs groupements, identiques ou différents, choisis parmi halogène et hydroxy, et/ou contenant éventuellement une ou plusieurs insaturations,
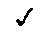 V représente un groupement choisi parmi atome d'hydrogène, d'halogène, groupement cyano, nitro, azido, alkyle (C₁-C₆) linéaire ou ramifié, aryle, arylalkyle (C₁-C₆) linéaire ou ramifié, hydroxy, alkoxy (C₁-C₆) linéaire ou ramifié, aryloxy, arylalkoxy (C₁-C₆) linéaire ou ramifié, formyle, carboxy, aminocarbonyle, NR₃R₄, -C(O)-T₁, -C(O)-NR₃-T_{1,} -NR₃-C(O)-T₁, -O-C(O)-T₁, -C(O)-O-T₁, -NR₃-T₂-NR₃R₄, -NR₃-T₂-OR₃, -NR₃-T₂-CO₂R₃, -O-T'₂-NR₃R₄, -O-T'₂-OR₃, -O-T'₂-CO₂R_{3,} et -S(O)₁-R₃,
dans lesquels :
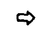 R₃ et R₄, identiques ou différents, représentent chacun un groupement choisi parmi atome d'hydrogène, groupement alkyle (C₁-C₆) linéaire ou ramifié, aryle, et arylalkyle (C₁-C₆) linéaire ou ramifié, ou R₃+R₄ forment ensemble, avec l'atome d'azote qui les portent, un hétérocycle de 5 à 10 atomes, monocyclique ou bicyclique, saturé, contenant éventuellement au sein du système cyclique un second hétéroatome choisi parmi oxygène et azote, et étant éventuellement substitué par un groupement choisi parmi alkyle (C₁-C₆) linéaire ou ramifié, aryle, arylalkyle (C₁-C₆) linéaire ou ramifié, hydroxy, alkoxy (C₁-C₆) linéaire ou ramifié, amino, monoalkylamino (C₁-C₆) linéaire ou ramifié, et dialkylamino (C₁-C₆) linéaire ou ramifié,
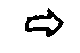 T₁ représente un groupement choisi parmi alkyle (C₁-C₆) linéaire ou ramifié, éventuellement substituée par un groupement choisi parmi -OR₃, -NR₃R₄, -CO₂R₃, -C(O)R₃ et -C(O)NR₃R₄ dans lesquels R₃ et R₄ sont tels que définis précédemment, aryle, arylalkyle (C₁-C₆) linéaire ou ramifié, ou T₁ représente une chaîne alkényle (C₂-C₆) linéaire ou ramifiée éventuellement substituée par un groupement choisi parmi -OR₃, -NR₃R₄, -CO₂R₃, -C(O)R₃ et -C(O)NR₃R₄ dans lesquels R₃ et R₄ sont tels que définis précédemment,
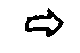 T₂ représente une chaîne alkylène (C₁-C₆) linéaire ou ramifiée,
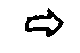 T'₂ représente une chaîne alkylène (C₁-C₆) linéaire ou ramifiée éventuellement substituée par un ou plusieurs groupements hydroxy,
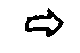 t représente un entier compris entre 0 et 2 inclus,
ou Z représente un groupement méthylènedioxy ou un groupement éthylènedioxy,
• **Q**_{**1**} représente un groupement choisi parmi atome d'oxygène ou un groupement NR₂ dans lequel R₂ représente un groupement choisi parmi atome d'hydrogène, groupement alkyle (C₁-C₆) linéaire ou ramifié, aryle, arylalkyle (C₁-C₆) linéaire ou ramifié, cycloalkyle, cycloalkylalkyle (C₁-C₆) linéaire ou ramifié, -OR₃, -NR₃R₄, -O-T₂-NR₃R₄, -NR₃-T₂-NR₃R₄, hydroxyalkylamino (C₁-C₆) linéaire ou ramifié, di(hydroxyalkyl) amino (C₁-C₆) linéaire ou ramifié, -C(O)-R₃, -NH-C(O)-R₃, ou une chaîne alkylène (C₁-C₆) linéaire ou ramifié substituée par un ou plusieurs groupements, identiques ou différents, choisis parmi atomes d'halogène, groupements cyano, nitro, -OR₃, -NR₃R₄, -CO₂R₃, -C(O)R₃, hydroxyalkylamino (C₁-C₆) linéaire ou ramifié, di(hydroxyalkyl)amino (C₁-C₆) linéaire ou ramifié, et -C(O)-NHR₃, les groupements R₃, R₄ et T₂ ayant les mêmes significations que précédemment,
• **Q**_{**2**} représente un groupement choisi parmi atome d'oxygène ou un groupement NR'₂ dans lequel R'₂ représente un groupement choisi parmi atome d'hydrogène, groupement alkyle (C₁-C₆) linéaire ou ramifié, aryle, arylalkyle (C₁-C₆) linéaire ou ramifié, cycloalkyle, cycloalkylalkyle (C₁-C₆) linéaire ou ramifié, -OR₃, -NR₃R₄, -O-T₂-NR₃R₄, -NR₃-T₂-NR₃R₄, hydroxyalkylamino (C₁-C₆) linéaire ou ramifié, di(hydroxyalkyl)amino (C₁-C₆) linéaire ou ramifié, -C(O)-R₃, -NH-C(O)-R₃, ou une chaîne alkylène (C₁-C₆) linéaire ou ramifié substituée par un ou plusieurs groupements, identiques ou différents, choisis parmi atomes d'halogène, groupements cyano, nitro, -OR₃, -NR₃R₄, -CO₂R₃, -C(O)R₃, hydroxyalkylamino (C₁-C₆) linéaire ou ramifié, di(hydroxyalkyl)amino (C₁-C₆) linéaire ou ramifié, et -C(O)-NHR₃, les groupements R₃, R₄ et T₂ ayant les mêmes significations que précédemment,
• **X**_{**1**} représente un groupement choisi parmi atome d'hydrogène, groupement hydroxy, alkoxy (C₁-C₆) linéaire ou ramifié, mercapto, et alkylthio (C₁-C₆) linéaire ou ramifié,
• **Y**_{**1**} représente un atome d'hydrogène, ou
• **X**_{**1**} et **Y**_{**1**} forment ensemble, avec l'atome de carbone qui les porte, un groupement carbonyle ou thiocarbonyle,
• **X**_{**2**} représente un groupement choisi parmi atome d'hydrogène, groupement hydroxy, alkoxy (C₁-C₆) linéaire ou ramifié, mercapto, et alkylthio (C₁-C₆) linéaire ou ramifié,
• **Y**_{**2**} représente un atome d'hydrogène, ou
• **X**_{**2**} et **Y**_{**2**} forment ensemble, avec l'atome de carbone qui les porte, un groupement carbonyle ou thiocarbonyle,
• **X'**_{**1**} représente un groupement choisi parmi atome d'hydrogène, groupement hydroxy, alkoxy (C₁-C₆) linéaire ou ramifié, mercapto, et alkylthio (C₁-C₆) linéaire ou ramifié,
• **Y'**_{**1**} représente un atome d'hydrogène, ou
• **X'**_{**1**} et **Y'**_{**1**} forment ensemble, avec l'atome de carbone qui les porte, un groupement carbonyle ou thiocarbonyle,
• **X'**_{**2**} représente un groupement choisi parmi atome d'hydrogène, groupement hydroxy, alkoxy (C₁-C₆) linéaire ou ramifié, mercapto, et alkylthio (C₁-C₆) linéaire ou ramifié,
• **Y'**_{**2**} représente un atome d'hydrogène, ou
• **X'**_{**2**} et **Y'**_{**2**} forment ensemble, avec l'atome de carbone qui les porte, un groupement carbonyle ou thiocarbonyle,
• **R**_{**1**} représente un groupement choisi parmi atome d'hydrogène, groupement alkyle (C₁-C₆) linéaire ou ramifié éventuellement substitué par un ou plusieurs groupements hydroxy, alkoxy (C₁-C₆) linéaire ou ramifié, hydroxyalkoxy (C₁-C₆) linéaire ou ramifié, NR₃R₄, les groupements R₃ et R₄ ayant les mêmes définitions que précédemment, ou R₁ représente un groupement de formule (a) : 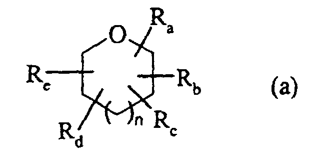 dans laquelle :
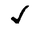 **R**_{**a**}**, R**_{**b**}**, R**_{**c**} **et R**_{**d**} identiques ou différents, indépendamment l'un de l'autre, représentent chacun une liaison ou un groupement choisi parmi atome d'hydrogène, atome d'halogène, groupement hydroxy, alkoxy (C₁-C₆) linéaire ou ramifié, aryloxy, arylalkoxy (C₁-C₆) linéaire ou ramifié, alkyle (C₁-C₆) linéaire ou ramifié, arylalkyle (C₁-C₆) linéaire ou ramifié, aryle, -NR₃R₄ dans lequel R₃ et R₄ sont tels que définis précédemment, azido, -N=NR₃ (dans lequel R₃ est tel que défini précédemment), et -O-C(O)-R₅ dans lequel R₅ représente un groupement alkyle (C₁-C₆) linéaire ou ramifié (éventuellement substitué par un ou plusieurs groupements choisis parmi halogène, hydroxy, amino, alkylamino (C₁-C₆) linéaire ou ramifié, et dialkylamino (C₁-C₆) linéaire ou ramifié), aryle, arylalkyle (C₁-C₆) linéaire ou ramifié, cycloalkyle, ou hétérocycloalkyle,
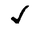 **R**_{**e**} représente un groupement méthylène (H₂C=) ou un groupement de formule -U₁-Rₐ dans laquelle U₁ représente une liaison simple ou un groupement méthylène, et Rₐ est tel que défini précédemment,
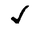 **n** prend la valeur 0 ou 1,
étant entendu que le groupement de formule (a) est lié à l'atome d'azote par Rₐ, R_{b}, R_{c}, R_{d} ou Rₑ,
leurs énantiomères, diastéréoisomères, ainsi que leurs sels d'addition à un acide ou à une base pharmaceutiquement acceptable,
étant entendu que les composés de formule (I) sont différents des composés suivants :
- 3b,6a,6b,7-tétrahydro-1*H*-dipyrrolo[3,4-a:3,4-c]carbazole-1,3,4,6(2*H*,3a*H*,5*H*)-tétrone ;
- 5-éthyl-3b,6a,6b,7-tétrahydro-1*H*-dipyrrolo[3,4-a:3,4-c]carbazole-1,3,4,6(2*H*,3a*H*,5*H*)-tétrone
- 3b,6a,7,11c-tétrahydro-1*H*-dipyrrolo[3,4-a:3,4-c]carbazole-1,3,4,6(2*H*,3a*H*,5*H*)-tétrone ;
- 3b,6a,6b,7-tétrahydrofuro[3,4-a]pyrrolo[3,4-c]carbazole-1,3,4,6(2*H*,3a*H*,5*H*)-tétrone ;
étant entendu que par aryle, on comprend un groupement phényle, naphtyle, dihydronaphtyle, tétrahydronaphtyle, indényle ou indanyle, chacun de ces groupements étant éventuellement substitué par un ou plusieurs groupements, identiques ou différents, choisis parmi halogène, alkyle (C₁-C₆) linéaire ou ramifié, trihalogénoalkyle (C₁-C₆) linéaire ou ramifié, hydroxy, alkoxy (C₁-C₆) linéaire ou ramifié, et NR₃R₄, R₃ et R₄ ayant les mêmes définitions que précédemment,

2. Composés de formule (I) selon la revendication 1 **caractérisés en ce que** X₁ et Y₁ forment ensemble avec l'atome de carbone qui le porte, un groupement carbonyle, X₂ et Y₂ forment ensemble avec l'atome de carbone qui le porte, un groupement carbonyle, X'₁ et Y'₁ forment ensemble avec l'atome de carbone qui le porte, un groupement carbonyle et X'₂ et Y'₂ forment ensemble avec l'atome de carbone qui le porte, un groupement carbonyle, leurs énantiomères, diastéréoisomères ainsi que leurs sels d'addition à un acide ou à une base pharmaceutiquement acceptable.

3. Composés de formule (I) selon l'une quelconque des revendications 1 à 2 **caractérisés en ce que** Q₁ représente un groupement -NR₂, dans lequel R₂ est tel que défini dans la formule (I), leurs énantiomères, diastéréoisomères ainsi que leurs sels d'addition à un acide ou à une base pharmaceutiquement acceptable.

4. Composés de formule (I) selon l'une quelconque des revendications 1 à 3 **caractérisés en ce que** Q₂ représente un groupement -NR'₂, dans lequel R'₂ est tel que défini dans la formule (I), leurs énantiomères, diastéréoisomères ainsi que leurs sels d'addition à un acide ou à une base pharmaceutiquement acceptable.

5. Composés de formule (I) selon l'une quelconque des revendications 1 à 4 **caractérisés en ce qu'**ils représentent des composés de formule (IA) : 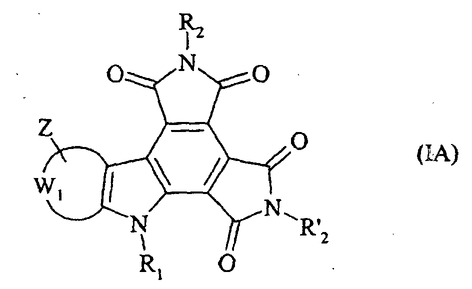 dans laquelle R₁, R₂, R'₂, W₁ et Z sont tels que définis dans la formule (I), leurs énantiomères, diastéréoisomères ainsi que leurs sels d'addition à un acide ou à une base pharmaceutiquement acceptable.

6. Composés de formule (I) selon l'une quelconque des revendications 1 à 5, **caractérisés en ce qu'**ils représentent des composés de formule (IB) : 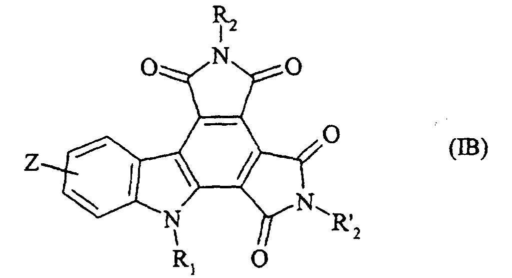 dans laquelle R₁, R₂, R'₂ et Z sont tels que définis dans la formule (I), leurs énantiomères, diastéréoisomères ainsi que leurs sels d'addition à un acide ou à une base pharmaceutiquement acceptable.

7. Composés de formule (I) selon l'une quelconque des revendications 1 à 5, **caractérisés en ce qu'**ils représentent des composés de formule (IC) : 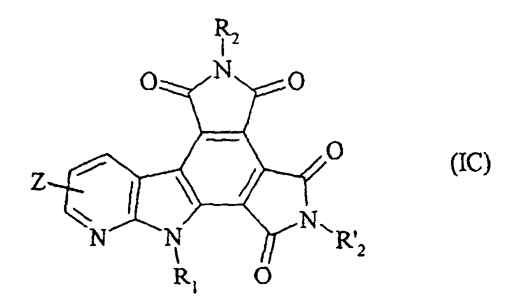 dans laquelle R₁, R₂, R'₂ et Z sont tels que définis dans la formale (I), leurs énantiomères, diastéréoisomères ainsi que leurs sels d'addition à un acide ou à une base pharmaceutiquement acceptable.

8. Composés de formule (I) selon l'une quelconque des revendications 1 à 4, **caractérisés en ce qu'**ils représentent des composés de formule (ID) : 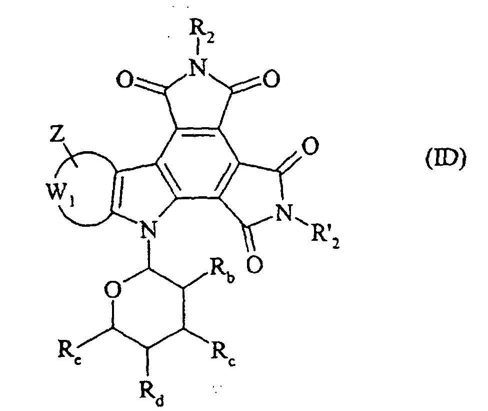 dans laquelle R₂, R'₂, W₁, Z, R_{b}, R_{c}, R_{d} et Rₑ sont tels que définis dans la formule (I), leurs énantiomères, diastéréoisomères ainsi que leurs sels d'addition à un acide ou à une base pharmaceutiquement acceptable.

9. Composés de formule (I) selon l'une quelconque des revendications 1 à 4 et 8, **caractérisés en ce qu'**ils représentent des composés de formule (IE) : 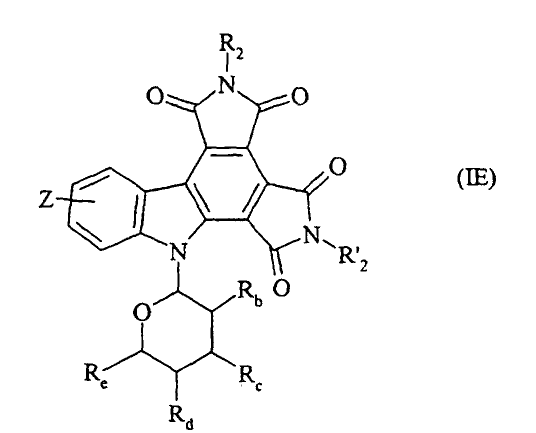 dans laquelle R₂, R'₂, Z, R_{b}, R_{c}, R_{d} et Rₑ sont tels que définis dans la formule (I), leurs énantiomères, diastéréoisomères ainsi que leurs sels d'addition à un acide ou à une base pharmaceutiquement acceptable.

10. Composés de formule (I) selon l'une quelconque des revendications 1 à 4 et 8, **caractérisés en ce qu'**ils représentent des composés de formule (IF) : 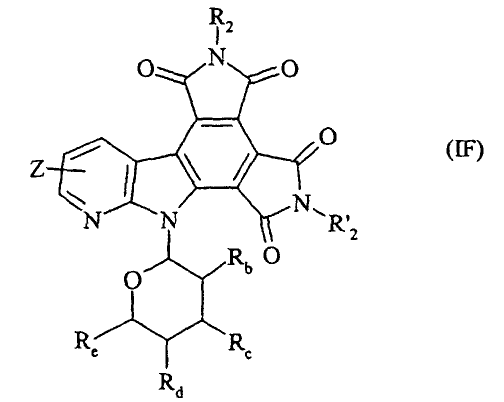 dans laquelle R₂, R'₂, Z, R_{b}, R_{c}, R_{d} et Rₑ sont tels que définis dans la formule (I), leurs énantiomères, diastéréoisomères ainsi que leurs sels d'addition à un acide ou à une base pharmaceutiquement acceptable.

11. Composés de formule (I) selon l'une quelconque des revendications 1 à 10, **caractérisés en ce que** Z représente le groupement de formule U-V dans laquelle U représente une liaison simple et V représente un groupement choisi parmi atome d'hydrogène, d'halogène, groupement nitro, alkyle (C₁-C₆) linéaire ou ramifié, hydroxy, alkoxy (C₁-C₆) linéaire ou ramifié, arylalkoxy (C₁-C₆) linéaire ou ramifié et NR₃R₄ dans lequel R₃ et R₄ representent chacun un atome d'hydrogène, leurs énantiomères, diastéréoisomères ainsi que leurs sels d'addition à un acide ou à une base pharmaceutiquement acceptable.

12. Composés de formule (I) selon l'une quelconque des revendications 1 à 11, **caractérisés en ce que** Z représente le groupement de formule U-V dans laquelle U représente une liaison simple et V représente un groupement choisi parmi atome d'hydrogène, halogène, groupement hydroxy et arylalkoxy (C₁-C₆) linéaire ou ramifié, leurs énantiomères, diastéréoisomères ainsi que leurs sels d'addition à un acide ou à une base pharmaceutiquement acceptable.

13. Composés de formule (I) selon l'une quelconque des revendications 1 à 12, **caractérisés en ce que** R₁ représente un atome d'hydrogène, le groupement alkyle (C₁-C₆) linéaire ou ramifié et le groupement de formule (a) : 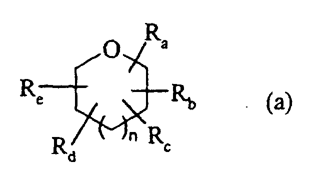 lié à l'atome d'azote par Ra
dans laquelle :
• R_{b}, R_{c,} et R_{d} représentent un groupement hydroxy, arylalkoxy (C₁-C₆) linéaire ou ramifié -O-C(O)-R₅ dans lequel R₅ représente un groupement alkyle (C₁-C₆) linéaire ou ramifié,
• Rₑ représente un groupement de formule U₁-Rₐ dans laquelle U₁ représente un groupement méthylène et Rₐ prend les mêmes définitions que R_{b}, R_{c} et R_{d} et n prend la valeur 0, leurs énantiomères, diastéréoisomères ainsi que leurs sels d'addition à un acide ou à une base pharmaceutiquement acceptable.

14. Composés de formule (I) selon l'une quelconque des revendications 1 à 13, **caractérisés en ce que** R₁ représente un atome d'hydrogène, leurs énantiomères, diastéréoisomères ainsi que leurs sels d'addition à un acide ou à une base pharmaceutiquement acceptable.

15. Composés de formule (I) selon l'une quelconque des revendications 1 à 14, **caractérisés en ce que** R₂ représente un atome d'hydrogène, un groupement alkyle (C₁-C₆) linéaire ou ramifié, OR₃, NR₃R₄ ou une chaîne alkylène (C₁-C₆) linéaire ou ramifié substituée par un groupement OR₃ ou NR₃R₄ dans lesquels R₃ et R₄ sont tels que définis dans la formule (I), leurs énantiomères, diastéréoisomères ainsi que leurs sels d'addition à un acide ou à une base pharmaceutiquement acceptable.

16. Composés de formule (I) selon l'une quelconque des revendications 1 à 15, **caractérisés en ce que** R₂ représente un atome d'hydrogène, un groupement alkyle (C₁-C₆) linéaire ou ramifié, ou une chaîne alkylène (C₁-C₆) linéaire ou ramifié substituée par un groupement NR₃R₄ dans lequel R₃ et R₄ sont tels que définis dans la formule (I), leurs énantiomères, diastéréoisomères ainsi que leurs sels d'addition à un acide ou à une base pharmaceutiquement acceptable.

17. Composés de formule (I) selon l'une quelconque des revendications 1 à 16, **caractérisés en ce que** R'₂ représente un atome d'hydrogène, un groupement alkyle (C₁-C₆) linéaire ou ramifié ou une chaîne alkylène (C₁-C₆) linéaire ou ramifié substituée par un groupement NR₃R₄ dans lequel R₃ et R₄ sont tels que définis dans la formule (I), leurs énantiomères, diastéréoisomères ainsi que leurs sels d'addition à un acide ou à une base pharmaceutiquement acceptable.

18. **-** Composés de formule (I) selon la revendication 1 qui sont le :
• 1*H*-dipyrrolo[3,4-a:3,4-c]carbazole-1,3,4,6(2*H*,5*H*,7*H*)-tétrone,
• 2-méthyl-1*H*-dipyrrolo[3,4-a:3,4-c]carbazole-1,3,4,6(2*H*,5*H*,7*H*)-tétrone,
• 2,5-diméthyl-1*H*-dipyrrolo[3,4-a:3,4-c]carbazole-1,3,4,6(2*H*,5*H*,7*H*)-tétrone,
• 2-[2-(diéthylamino)éthyl]-5-méthyl-1*H*-dipyrrolo[3,4-a:3,4-c]carbazole-1,3,4,6 (2*H*,5*H*,7*H*)-tétrone,
• 10-hydroxy-1*H*-dipyrrolo[3,4-a:3,4-c]carbazole-1,3,4,6(2*H*,5*H*,7*H*)-tétrone

19. Procédé de préparation des composés de formule (I), selon la revendication 1,
**caractérisé en ce qu'**on utilise comme produit de départ un composé de formule (II) : 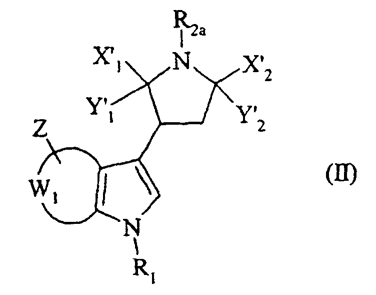 dans laquelle R₂ₐ représente un atome d'hydrogène ou un groupement méthyle et, R₁, X'₁, Y'₁, X'₂, Y'₂, W₁ et Z sont tels que définis dans la formule (I),
qui est traité avec du 2,3-dichloro-5,6-dicyano-1,4-benzoquinone pour conduire au composé de formule (III) : 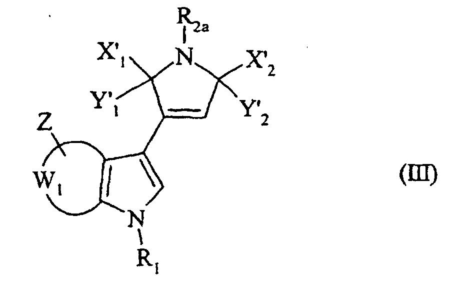 dans laquelle R₁, R₂ₐ, X'₁, Y'₁, X'₂, Y'₂, W₁ et Z sont tels que définis précédemment,
composé de formule (III) qui est :
* *soit* traité par de la soude aqueuse puis placé en présence d'acide chlorhydrique pour conduire au composé de formule (IV) 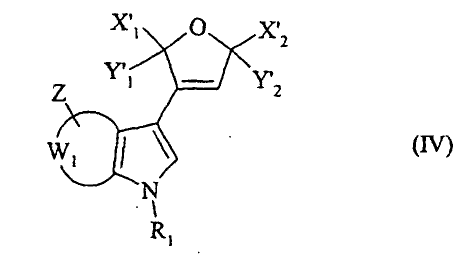 dans laquelle R₁, X'₁, Y'₁, X'₂, Y'₂, W₁ et Z sont tels que définis précédemment,
composé de formule (IV) qui est traité par un composé de formule (V) : 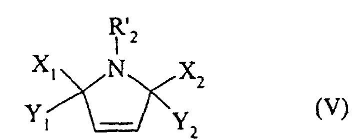 dans laquelle R'₂, X₁, Y₁, X₂ et Y₂ sont tels que définis dans la formule (I) pour conduire au composé de formules (I/a) et (I/b), cas particulier des composés de formule (I) : 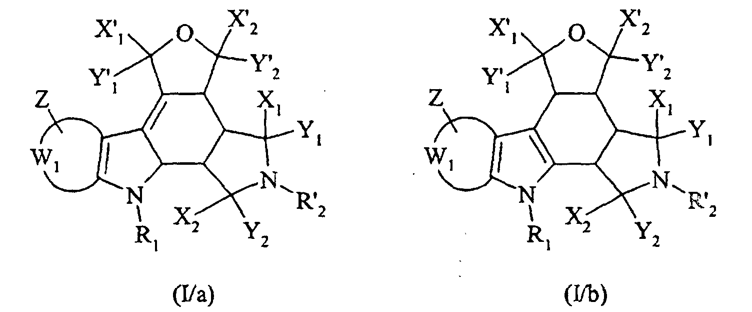 dans laquelle R₁, R'₂, X₁, Y₁, X₂, Y₂, X'₁, Y'₁, X'₂, Y'₂, W₁ et Z sont tels que définis précédemment,
composés de formule (I/a) et/ou (I/b) qui sont éventuellement soumis à l'action d'acide trifluoroacétique pour conduire au composé de formule (I/c), cas particulier des composés de formule (I) : 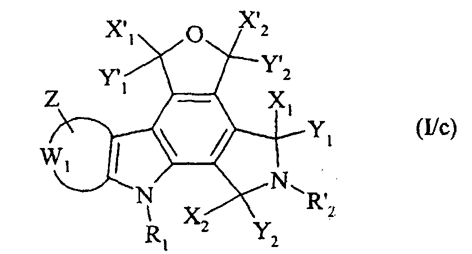 dans laquelle R₁, R'₂, X₁, Y₁, X₂, Y₂, X'₁, Y'₁, X'₂, Y'₂, W₁ et Z sont tels que définis précédemment,
l'ensemble des composés de formules (I/a), (I/b) et (I/c) forment les composés de formule (I/d) : 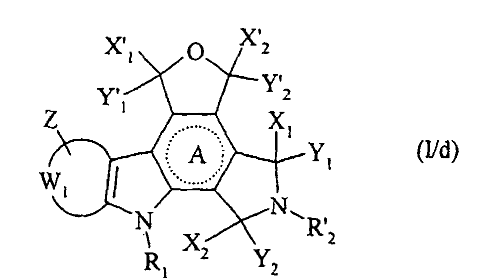 dans laquelle A, R₁, R'₂, X₁, Y₁, X₂, Y₂, X'₁, Y'₁, X'₂, Y'₂, W₁ et Z sont tels que définis précédemment,
composé de formule (I/d) qui est éventuellement soumis à l'action d'un composé de formule (VII) :
R_{2b} - NH₂ (VII)
dans laquelle R_{2b} a la même définition que R₂, dans la formule (I), à l'exception des définitions atome d'hydrogène et groupement méthyle, pour conduire aux composés de formule (I/e), cas particulier des composés de formule (I) : 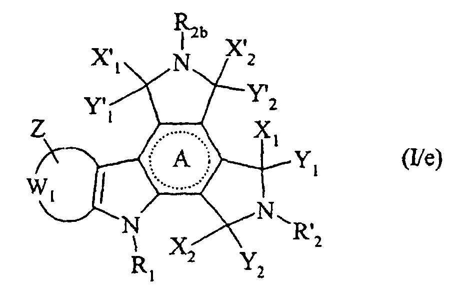 dans laquelle A, R₁, R'₂, R_{2b}, X₁, Y₁, X₂, Y₂, X'₁, Y'₁, X'₂, Y'₂, W₁ et Z sont tels que définis précédemment,
* *soit* soumis successivement aux mêmes conditions de réaction que les composés de formules (IV), (I/a) et (I/b), pour conduire au composé de formule (I/f), cas particulier des composés de formule (I) : 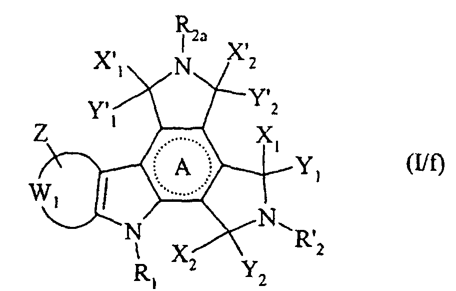 dans laquelle A, R₁, R'₂, R₂ₐ, X₁, Y₁, X₂, Y₂, X'₁, Y'₁, X'₂, Y'₂, W₁ et Z sont tels que définis précédemment,
l'ensemble des composés (I/d), (I/e) et (I/f) formant les composés de formule (I/g) : 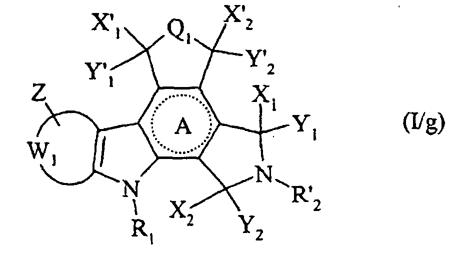 dans laquelle A, R₁, R'₂, Q₁, X₁, Y₁, X₂, Y₂, X'₁, Y'₁, X'₂, Y'₂, W₁ et Z sont tels que définis précédemment,
composé de formule (I/g) qui, dans le cas particulier où R'₂ représente un atome d'hydrogène ou un groupement méthyle, est éventuellement soumis successivement aux mêmes conditions de réaction que le composé de formule (III) pour conduire au composé de formule (I/i), cas particulier des composés de formule (I) : 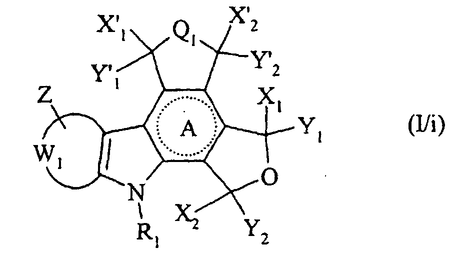 dans laquelle A, R₁, Q₁, X₁, Y₁, X₂, Y₂, X'₁, Y'₁, X'₂, Y'₂, W₁ et Z sont tels que définis précédemment,
composé de formule (I/i) qui est éventuellement soumis à l'action d'un composé (VIII) :
R'_{2b} - NH₂ (VIII)
dans laquelle R'_{2b} a la même définition que R'₂ dans la formule (I), à l'exception des définitions atome d'hydrogène et groupement méthyle, pour conduire aux composés de formule (I/j), cas particulier des composés de formule (I) : 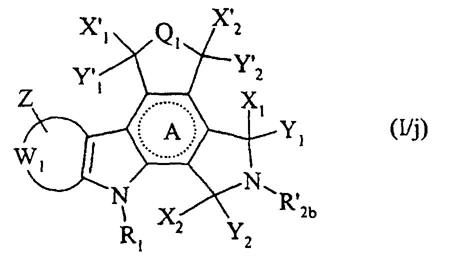 dans laquelle A, R₁, R'_{2b}, Q₁, X₁, Y₁, X₂, Y₂, X'₁, Y'₁, X'₂, Y'₂, W₁ et Z sont tels que définis précédemment,
les composés de formule (I/a) à (I/j) formant l'ensemble des composés de formule (I), que l'on purifie, le cas échéant, selon des techniques classiques de purification, qui peuvent, si on le désire, être séparés en leurs différents isomères, selon une technique classique de séparation, dont on module les substituants Rₐ, R_{b}, R_{c}, R_{d} et Rₑ selon les méthodes classiques de la synthèse organique utilisées dans le domaine de la chimie des sucres, et que l'on transforme, si on le souhaite, en leurs sels d'addition à un acide ou à une base pharmaceutiquement acceptable.

20. Compositions pharmaceutiques contenant comme principe actif au moins un composé de formule (I) selon l'une quelconque des revendications 1 à 18, seul ou en combinaison avec un ou plusieurs excipients ou véhicules inertes, non toxiques, pharmaceutiquement acceptables.

21. Compositions pharmaceutiques selon la revendication 20, utiles en tant que médicament, dans le traitement des cancers.

## Patentansprüche

1. Verbindungen der Formel (I): 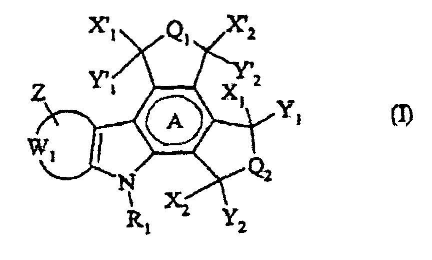 in der
• **A** einen Ring bedeutet, der gesättigt, teilweise oder vollständig ungesättigt ist und dem Ring gegebenenfalls einen aromatischen Charakter verleiht,
• **W**_{**1**} zusammen mit den Kohlenstoffatomen, an die es gebunden ist, eine Phenylgruppe oder eine Pyridinylgruppe bedeutet,
• **Z** eine oder mehrere gleichartige oder verschiedenartige Gruppen der Formel U-V bedeutet, worin:
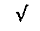 U eine Einfachbindung, eine geradkettige oder verzweigte (C₁-C₆)-Alkylenkette, eine geradkettige oder verzweigte (C₂-C₆)-Alkenylkette, die gegebenenfalls durch eine oder mehrere gleichartige oder verschiedenartige Gruppen ausgewählt aus Halogen und Hydroxy substituiert ist und/oder gegebenenfalls eine oder mehrere Unsättigungen aufweist, bedeutet,
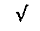 V eine Gruppe bedeutet, ausgewählt aus dem Wasserstoffatom, Halogenatomen, Cyano-, Nitro-, Azido-, geradkettigen oder verzweigten (C₁-C₆)-Alkyl-, -Aryl-, geradkettigen oder verzweigten Aryl-(C₁-C₆)-alkyl-, Hydroxy-, geradkettigen oder verzweigten (C₁-C₆)-Alkoxy-, Aryloxy-, geradkettigen oder verzweigten Aryl-(C₁-C₆)-alkoxy-, Formyl-, Carboxy-, Aminocarbonylgruppen oder Gruppen der Formeln NR₃R₄, -C(O)-T₁, -C(O)-NR₃-T₁, -NR₃-C(O)-T₁, -O-C(O)-T₁, -C(O)-O-T₁, -NR₃-T₂-NR₃R₄, -NR₃-T₂-OR₃. -NR₃-T₂-CO₂R₃, -O-T'₂-NR₃R₄, -O-T'₂-OR₃, -O-T'₂-CO₂R₃ und -S(O)ₜ-R₃,
in denen:
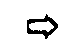 R₃ und R₄, die gleichartig oder verschieden sind, jeweils eine Gruppe bedeuten ausgewählt aus dem Wasserstoffatom, geradkettigen oder verzweigten (C₁-C₆)-Alkyl-, Aryl- und geradkettigen oder verzweigten Aryl-(C₁-C₆)-alkylgruppen, oder R₃ + R₄ gemeinsam mit dem sie tragenden Stickstoffatom eine monocyclischen oder bicyclischen gesättigten Heterocyclus mit 5 bis 10 Kohlenstoffatomen bilden, welcher gegebenenfalls in dem cyclischen System ein zweites Heteroatom aufweist, ausgewählt aus Sauerstoff und Stickstoff, und welcher gegebenenfalls durch eine Gruppe substituiert ist ausgewählt aus geradkettigem oder verzweigtem (C₁-C₆)-Alkyl, Aryl, geradkettigem oder verzweigtem Aryl-(C₁-C₆)-alkyl, Hydroxy, geradkettigem oder verzweigtem (C₁-C₆)-Alkoxy, Amino, geradkettigem oder verzweigtem (C₁-C₆)-Monoalkylamino und geradkettigem oder verzweigtem Di-(C₁-C₆)-alkylamino,
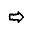 T₁ eine Gruppe bedeutet ausgewählt aus geradkettigem oder verzweigtem (C₁-C₆)-Alkyl, welches gegebenenfalls substituiert ist durch eine Gruppe ausgewählt aus -OR₃, -NR₃R₄, -CO₂R₃, -C(O)R₃ und -C(O)NR₃R₄, in denen R₃ und R₄ die oben angegebenen Bedeutungen besitzen, Aryl, geradkettigem oder verzweigtem Aryl-(C₁-C₆)-alkyl, oder T₁ eine geradkettige oder verzweigte (C₂-C₆)-Alkenylkette bedeutet, welche gegebenenfalls durch eine Gruppe ausgewählt aus -OR₃, -NR₃R₄, -CO₂R₃, -C(O)R₃ und -C(O)NR₃R₄ substituiert ist, in denen R₃ und R₄ die oben angegebenen Bedeutungen besitzen,
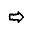 T₂ eine geradkettige oder verzweigte (C₁-C₆)-Alkylenkette bedeutet,
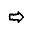 T'₂ eine geradkettige oder verzweigte (C₁-C₆)-Alkylenkette bedeutet, welche gegebenenfalls durch eine oder mehrere Hydroxygruppen substituiert ist,
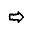 t eine ganze Zahl mit einem Wert zwischen 0 und 2 einschließlich bedeutet,
oder Z eine Methylendioxygruppe oder eine Ethylendioxygruppe darstellt,
• **Q**_{**1**} eine Gruppe bedeutet ausgewählt aus dem Sauerstoffatom oder einer Gruppe NR₂, in der R₂ eine Gruppe bedeutet ausgewählt aus dem Wasserstoffatom, geradkettigen oder verzweigten (C₁-C₆)-Alkyl-, Aryl-, geradkettigen oder verzweigten Aryl-(C₁-C₆)-alkyl-, Cycloalkyl-, geradkettigen oder verzweigten (C₁-C₆)-Cycloalkylalkyl-, -OR₃, -NR₃R₄, -O-T₂-NR₃R₄, -NR₃T₂-NR₃R₄, geradkettigen oder verzweigten (C₁-C₆)-Hydroxyalkylamino-, geradkettigen oder verzweigten Di-(C₁-C₆)-(hydroxyalkyl)-amino-gruppen, -C(O)-R₃, -NH-C(O)-R₃, oder einer geradkettigen oder verzweigten (C₁-C₆)-Alkylenkette, welche durch eine oder mehrere gleichartige oder verschiedenartige Gruppen substituiert ist, ausgewählt aus Halogenatomen, Cyano-, Nitro-, -OR₃, -NR₃R₄, -CO₂R₃, -C(O)R₃, geradkettigen oder verzweigten (C₁-C₆)-Hydroxyalkylamino-, geradkettigen oder verzweigten Di-((C₁-C₆)-hydroxyalkyl)-amino-gruppen und -C(O)-NHR₃, wobei die Gruppen R₃, R₄ und T₂ die oben angegebenen Bedeutungen besitzen,
• **Q**_{**2**} eine Gruppe bedeutet ausgewählt aus dem Sauerstoffatom oder einer Gruppe NR'₂, in der R'₂ eine Gruppe bedeutet, ausgewählt aus dem Wasserstoffatom, geradkettigen oder verzweigten (C₁-C₆)-Alkyl-, Aryl-, geradkettigen oder verzweigten (C₁-C₆)-Arylalkyl-, Cycloalkyl-, geradkettigen oder verzweigten (C₁-C₆)-Cycloalkylalkyl-, -OR₃, -NR₃R₄, -O-T₂-NR₃R₄, -NR₃-T₂-NR₃R₄, geradkettigen oder verzweigten (C₁-C₆)-Hydroxyalkylamino-, geradkettigen oder verzweigten Di-(C₁-C₆)-(hydroxyalkyl)-amino-gruppen, -C[O]-R₃, -NH-C(O)-R₃, oder einer geradkettigen oder verzweigten (C₁-C₆)-Alkylenkette, die durch eine oder mehrere, gleichartige oder verschiedenartige Gruppen substituiert ist, ausgewählt aus Halogenatomen, Cyano-, Nitro-, -OR₃, -NR₃R₄, -CO₂R₃ -C(O)R₃, geradkettigen oder verzweigten (C₁-C₆)-Hydroxyalkylamino-, geradkettigen oder verzweigten Di-(C₁-C₆)-(hydroxyalkyl)-amino-gruppen und -C(O)-NHR₃, wobei die Gruppen R₃, R₄ und T₂ die oben angegebenen Bedeutungen besitzen,
• **X**_{**1**} eine Gruppe bedeutet ausgewählt aus dem Wasserstoffatom, Hydroxy-, geradkettigen oder verzweigten (C₁-C₆)-Alkoxy-, Mercapto- und geradkettigen oder verzweigten (C₁-C₆)-Alkylthio-gruppen,
• **Y**_{**1**} ein Wasserstoffatom bedeutet, oder
• **X**_{**1**} und **Y**_{**1**} gemeinsam mit dem sie tragenden Kohlenstoffatom eine Carbonyloder Thiocarbonylgruppe bilden,
• **X**_{**2**} eine Gruppe bedeutet ausgewählt aus dem Wasserstoffatom, Hydroxy-, geradkettigen oder verzweigten (C₁-C₆)-Alkoxy-, Mercapto- und geradkettigen oder verzweigten (C₁-C₆)-Alkylthiogruppen,
• **Y**_{**2**} ein Wasserstoffatom bedeutet, oder
• **X**_{**2**} und **Y**_{**2**} gemeinsam mit dem sie tragenden Kohlenstoffatom eine Carbonyloder Thiocarbonylgruppe bilden,
• **X'**_{**1**} eine Gruppe bedeutet ausgewählt aus dem Wasserstoffatom, Hydroxy-, geradkettigen oder verzweigten (C₁-C₆)-Alkoxy-, Mercapto- und geradkettigen oder verzweigten (C₁-C₆)-Alkylthiogruppen,
• **Y'**_{**1**} ein Wasserstoffatom bedeutet, oder
• **X'**_{**1**} und **Y'**_{**1**} gemeinsam mit dem sie tragenden Kohlenstoffatom eine Carbonyl- oder Thiocarbonylgruppe bilden,
• **X'**_{**2**} eine Gruppe bedeutet ausgewählt aus dem Wasserstoffaatom, Hydroxy-, geradkettigen oder verzweigten (C₁-C₆)-Alkoxy-, Mercapto- und geradkettigen oder verzweigten (C₁-C₆)-Alkylthiogruppen,
• **Y'**_{**2**} ein Wasserstoffatom bedeutet, oder
• **X'**_{**2**} und **Y'**_{**2**} gemeinsam mit dem sie tragenden Kohlenstoffatom eine Carbonyl- oder Thiocarbonylgruppe bilden,
• **R**_{**1**} eine Gruppe bedeutet ausgewählt aus dem Wasserstoffatom, geradkettigen oder verzweigten (C₁-C₆)-Alkyl-, welches gegebenenfalls substituiert ist durch eine oder mehrere Hydroxygruppen, geradkettigen oder verzweigten (C₁-C₆)-Alkoxy-, geradkettigen oder verzweigten (C₁-C₆)-Hydroxyalkoxy-gruppen, Gruppen NR₃R₄, worin die Gruppen R₃ und R₄ die oben angegebenen Bedeutungen besitzen, oder R₁ eine Gruppe der Formel (a) bedeutet: 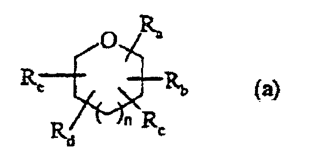 in der:
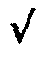 **R**_{**a,**} **R**_{**b,**} **R**_{**c**} **und R**_{**d,**} die gleichartig oder verschieden sind, unabhängig voneinander jeweils eine Bindung oder eine Gruppe bedeuten ausgewählt aus Wasserstoff- oder Halogenatomen, Hydroxy-, geradkettigen oder verzweigten (C₁-C₆)-Alkoxy-, Aryloxy-, geradkettigen oder verzweigten Aryl-(C₁-C₆)-alkoxy-, geradkettigen oder verzweigten (C₁-C₆)-Alkyl-, geradkettigen oder verzweigten Aryl-(C₁-C₆)-alkyl-, Aryl-, -NR₃R₄, in der R₃ und R₄ die oben angegebenen Bedeutungen besitzen. Azido-, -N=NR₃ (worin R₃ die oben angegebenen Bedeutungen besitzt) und -O-C(O)-R₅, in der R₅ eine geradkettige oder verzweigte (C₁-C₆)-Alkyl- (die gegebenenfalls durch eine oder mehrere Gruppen ausgewählt aus Halogen, Hydroxy, Amino, geradkettigem oder verzweigtem (C₁-C₆)-Alkylamino und geradkettigem oder verzweigtem Di-(C₁-C₆)-alkylamino substituiert ist). Aryl-, geradkettige oder verzweigte Aryl-(C₁-C₆)-alkyl-, Cycloalkyl- oder Heterocycloalkylgruppe darstellt,
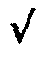 **R**_{**e**} eine Methylengruppe (H₂C=) oder eine Gruppe der Formel -U₁-Rₐ bedeutet, in der U₁ eine Einfachbindung oder eine Methylengruppe darstellt und Rₐ die oben angegebenen Bedeutungen besitzt,
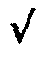 **n** den Wert 0 oder 1 aufweist,
wobei es sich versteht, daß die Gruppe der Formel (a) über Rₐ, R_{b}, R_{c}, R_{d} oder Rₑ an das Stickstoffatom gebunden ist,
deren Enantiomere, Diastereoisomere sowie deren Additionssalze mit einer pharmazeutisch annehmbaren Säure oder Base,
mit der Maßgabe, daß die Verbindungen der Formel (I) verschieden sind von den folgenden Verbindungen:
- 3b,6a,6b,7-Tetrahydro-1*H*-dipyrrolo[3,4-a:3,4-c]carbazol-1,3,4,6(2*H*,3a*H*,5*H*)-tetron;
- 5-Ethyl-3b,6a,6b,7-tetrahydro-1*H*-dipyrrolo[3,4-a:3,4c]carbazol-1,3,4,6-(2*H*,3a*H*,5*H*)-tetron;
- 3b,6a,7,11c-Tetrahydro-1*H*-dipyrrolo[3,4-a:3,4-c]carbazol-1,3,4,6(2*H*,3a*H*,5*H*)-tetron;
- 3b,6a,6b,7-Tetrahydrofuro[3,4-a]pyrrolo[3,4-c]carbazol-1,3,4,6(2*H*,3aH*,*5*H*)-tetron;
mit der Maßgabe, daß man unter Aryl eine Phenyl-, Naphthyl-, Dihydronaphthyl-, Tetrahydronaphthyl-, Indenyl- oder Indanyl-gruppe versteht, wobei jede dieser Gruppen gegebenenfalls durch eine oder mehrere gleichartige oder verschiedenartige Gruppen substituiert ist ausgewählt aus Halogen, geradkettigem oder verzweigtem (C₁-C₆)-Alkyl, geradkettigem oder verzweigtem (C₁-C₆)-Trihalogenalkyl, Hydroxy. geradkettigem oder verzweigtem (C₁-C₆)-Alkoxy und NR₃R₄, worin R₃ und R₄ die oben angegebenen Bedeutungen besitzen.

2. Verbindungen der Formel (I) nach Anspruch 1, **dadurch gekennzeichnet, daß** X₁ und Y₁ gemeinsam mit dem sie tragenden Kohlenstoffatom eine Carbonylgruppe bilden, X₂ und Y₂ gemeinsam mit dem sie tragenden Kohlenstoffatom eine Carbonylgruppe bilden, X'₁ und Y'₁ gemeinsam mit dem sie tragenden Kohlenstoffatom eine Carbonylgruppe bilden und X'₂ und Y'₂ gemeinsam mit dem sie tragenden Kohlenstoffatom eine Carbonylgruppe bilden, deren Enantiomere, Diastereoisomere sowie deren Additionssalze mit einer pharmazeutisch annehmbaren Säure oder Base.

3. Verbindungen der Formel (I) nach einem der Ansprüche 1 bis 2, **dadurch gekennzeichnet, daß** Q₁ eine Gruppe -NR₂ darstellt, in der R₂ die bezüglich der Formel (I) angegebenen Bedeutungen besitzt, deren Enantiomere, Diastereoisomere sowie deren Additionssalze mit einer pharmazeutisch annehmbaren Säure oder Base.

4. Verbindungen der Formel (I) nach einem der Ansprüche 1 bis 3, **dadurch gekennzeichnet, daß** Q₂ eine Gruppe -NR'₂ bedeutet, in der R'₂ die bezüglich der Formel (I) angegebenen Bedeutungen besitzt, deren Enantiomere, Diastereoisomere sowie deren Additionssalze mit einer pharmazeutisch annehmbaren Säure oder Base.

5. Verbindungen der Formel (I) nach einem der Ansprüche 1 bis 4, **dadurch gekennzeichnet, daß** sie die Verbindungen der Formel (LA) sind: 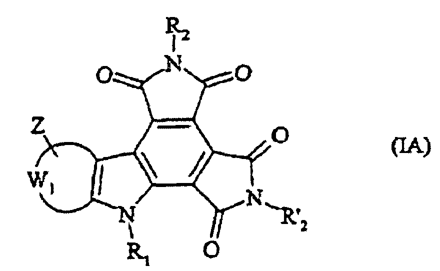 in der R₁, R₂, R'₂. W₁ und Z die bezüglich der Formel (I) angegebenen Bedeutungen besitzen, deren Enantiomere, Diastereoisomere sowie deren Additionssalze mit einer pharmazeutisch annehmbaren Säure oder Base.

6. Verbindungen der Formel (I) nach einem der Ansprüche 1 bis 5, **dadurch gekennzeichnet, daß** sie die Verbindungen der Formel (IB) sind: 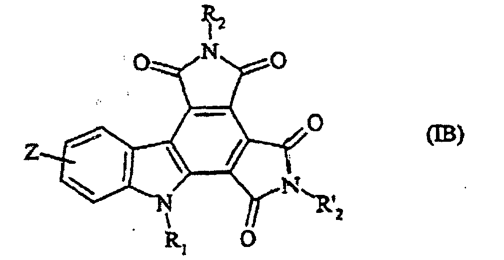 in der R₁, R₂, R'₂ und Z die bezüglich der Formel (I) angegebenen Bedeutungen besitzen, deren Enantiomere, Diastereoisomere sowie deren Additionssalze mit einer pharmazeutisch annehmbaren Säure oder Base.

7. Verbindungen der Formel (I) nach einem der Ansprüche 1 bis 5, **dadurch gekennzeichnet, daß** sie die Verbindungen der Formel (IC) sind: 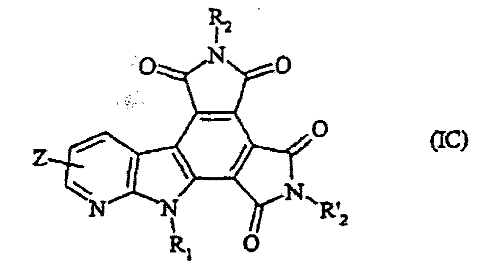 in der R₁, R₂, R'₂ und Z die bezüglich der Formel (I) angegebenen Bedeutungen besitzen, deren Enantiomere, Diastereoisomere sowie deren Additionssalze mit einer pharmazeutisch annehmbaren Säure oder Base.

8. Verbindungen der Formel (I) nach einem der Ansprüche 1 bis 4, **dadurch gekennzeichnet, daß** sie die Verbindungen der Formel (ID) sind: 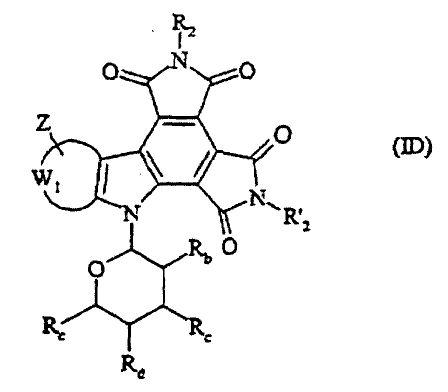 in der R₂, R'₂, W₁, Z, R_{b}, R_{c}, R_{d} und Rₑ die bezüglich der Formel (I) angegebenen Bedeutungen besitzen, deren Enantiomere, Diastereoisomere sowie deren Additionssalze mit einer pharmazeutisch annehmbaren Säure oder Base.

9. Verbindungen der Formel (I) nach einem der Ansprüche 1 bis 4 und 8, **dadurch gekennzeichnet, daß** sie die Verbindungen der Formel (IE) sind: 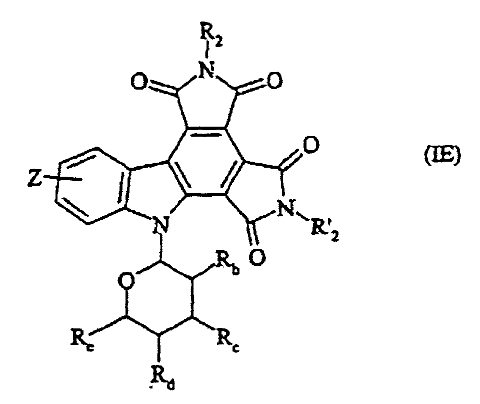 in der R₂, R'₂, Z, R_{b}, R_{c}, R_{d} und Rₑ die bezüglich der Formel (I) angegebenen Bedeutungen besitzen, deren Enantiomere, Diastereoisomere sowie deren Additionssalze mit einer pharmazeutisch annehmbaren Säure oder Base.

10. Verbindungen der Formel (I) nach einem der Ansprüche 1 bis 4 und 8, **dadurch gekennzeichnet, daß** sie die Verbindungen der Formel (IF) sind: 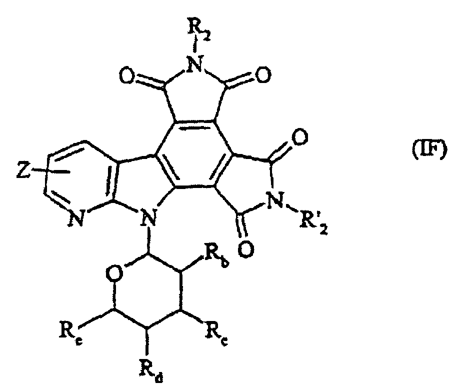 in der R₂, R'₂, Z, R_{b}, R_{c}, R_{d} und Rₑ die bezüglich der Formel (I) angegebenen Bedeutungen besitzen, deren Enantiomere, Diastereoisomere sowie deren Additionssalze mit einer pharmazeutisch annehmbaren Säure oder Base.

11. Verbindungen der Formel (I) nach einem der Ansprüche 1 bis 10, **dadurch gekennzeichnet, daß** Z die Gruppe der Formel U-V bedeutet, in der U eine Einfachbindung darstellt und V eine Gruppe bedeutet ausgewählt aus dem Wasserstoffatom, Halogenatomen, Nitro-, geradkettigen oder verzweigten (C₁-C₆)-Alkyl-, Hydroxy-, geradkettigen oder verzweigten (C₁-C₆)-Alkoxy-, geradkettigen oder verzweigten Aryl-(C₁-C₆)-alkoxygruppen und Gruppen NR₃R₄, in der R₃ und R₄ ein Wasserstoffatom bedeuten, deren Enantiomere, Diastereoisomere sowie deren Additionssalze mit einer pharmazeutisch annehmbaren Säure oder Base.

12. Verbindungen der Formel (I) nach einem der Ansprüche 1 bis 11, **dadurch gekennzeichnet, daß** Z die Gruppe der Formel U-V bedeutet, in der U eine Einfachbindung darstellt und V eine Gruppe bedeutet ausgewählt aus Wasserstoffatomen, Halogenatomen, Hydroxy- und geradkettigen oder verzweigten Aryl-(C₁-C₆)-alkoxygruppen, deren Enantiomere, Diastereoisomere sowie deren Additionssalze mit einer pharmazeutisch annehmbaren Säure oder Base.

13. Verbindungen der Formel (I) nach einem der Ansprüche 1 bis 12, **dadurch gekennzeichnet, daß** R₁ ein Wasserstoffatom, eine geradkettige oder verzweigte (C₁-C₆)-Alkylgruppe oder eine Gruppe der Formel (a) bedeutet: 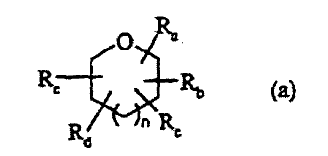 welche über Ra an das Stickstoffatom gebunden ist,
in der:
• R_{b}, R_{c} und R_{d} eine Hydroxy-, geradkettige oder verzweigte Aryl-(C₁-C₆)-alkoxygruppe oder eine Gruppe der Formel -O-C(O)-R₅, in der R₅ eine geradkettige oder verzweigte (C₁-C₆)-Alkylgruppe darstellt, bedeuten,
• Rₑ eine Gruppe der Formel U₁-Rₐ darstellt, in der U₁ eine Methylengruppe bedeutet und Rₐ die gleichen Bedeutungen besitzt wie R_{b}, R_{c} und R_{d} und worin n den Wert 0 besitzt, deren Enantiomere, Diastereoisomere sowie deren Additionssalze mit einer pharmazeutisch annehmbaren Säure oder Base.

14. Verbindungen der Formel (I) nach einem der Ansprüche 1 bis 13, **dadurch gekennzeichnet, daß** R₁ ein Wasserstoffatom bedeutet, deren Enantiomere, Diastereoisomere sowie deren Additionssalze mit einer pharmazeutisch annehmbaren Säure oder Base.

15. Verbindungen der Formel (I) nach einem der Ansprüche 1 bis 14, **dadurch gekennzeichnet, daß** R₂ ein Wasserstoffatom, eine geradkettige oder verzweigte (C₁-C₆)-Alkylgruppe. OR₃, NR₃R₄ oder eine geradkettige oder verzweigte (C₁-C₆)-Alkylenkette, die durch eine Gruppe OR₃ oder NR₃R₄ substituiert ist, in denen R₃ und R₄ die bezüglich der Formel (I) angegebenen Bedeutungen besitzen, bedeutet, deren Enantiomere, Diastereoisomere sowie deren Additionssalze mit einer pharmazeutisch annehmbaren Säure oder Base.

16. Verbindungen der Formel (I) nach einem der Ansprüche 1 bis 15, **dadurch gekennzeichnet, daß** R₂ ein Wasserstoffatom, eine geradkettige oder verzweigte (C₁-C₆)-Alkylgruppe oder eine geradkettige oder verzweigte (C₁-C₆)-Alkylenkette, die durch eine Gruppe NR₃R₄ substituiert ist, in der R₃ und R₄ die bezüglich der Formel (I) angegebenen Bedeutungen besitzen, bedeutet, deren Enantiomere, Diastereoisomere sowie deren Additionssalze mit einer pharmazeutisch annehmbaren Säure oder Base.

17. Verbindungen der Formel (I) nach einem der Ansprüche 1 bis 16, **dadurch gekennzeichnet, daß** R'₂ ein Wasserstoffatom, eine geradkettige oder verzweigte (C₁-C₆)-Alkylgruppe oder eine geradkettige oder verzweigte (C₁-C₆)-Alkylenkette, die durch eine Gruppe NR₃R₄ substituiert ist, in der R₃ und R₄ die bezüglich der Formel (I) angegebenen Bedeutungen besitzen, darstellt, deren Enantiomere, Diastereoisomere sowie deren Additionssalze mit einer pharmazeutisch annehmbaren Säure oder Base.

18. Verbindungen der Formel (I) nach Anspruch 1, nämlich:
• 1*H*-Dipyrrolo[3,4-a:3,4c]carbazol-1,3,4,6(2*H*,5*H*,7*H*)-tetron,
• 2-Methyl-1*H*-dipyrrolo[3,4-a:3,4c]carbazol-1,3,4,6(2*H*,5*H*,7*H*)-tetron,
• 2,5-Dimethyl-1*H*-dipyrrolo[3,4-a:3,4c]carbazol-1,3,4,6(2*H*,5*H*,7*H*)-tetron,
• 2-]2-(Diethylamino)-ethyl]-5-methyl-1*H*-dipyrrolo[3,4-a:3,4c]carbazol-1,3,4,6-(2*H*,5*H*,7*H*)-tetron,
• 10-Hydroxy-1*H*-dipyrrolo[3,4-a:3,4c]carbazol-1,3,4,6(2*H*,5*H*,7*H*)-tetron.

19. Verfahren zur Herstellung der Verbindungen der Formel (I) nach Anspruch 1, **dadurch gekennzeichnet, daß** man als Ausgangsprodukt eine Verbindung der Formel (II) verwendet: 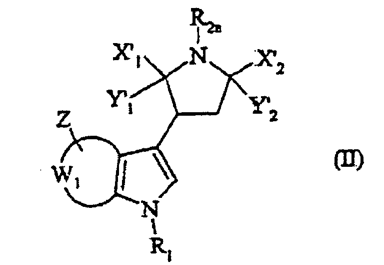 in der R₂ₐ ein Wasserstoffatom oder eine Methylgruppe bedeutet und R₁, X'₁, Y'₁, X'₂, Y'₂, W₁ und Z die bezüglich der Formel (I) angegebenen Bedeutungen besitzen, welche man mit 2,3-Dichlor-5,6-dicyano-1,4-benzochinon behandelt zur Bildung der Verbindung der Formel (III): 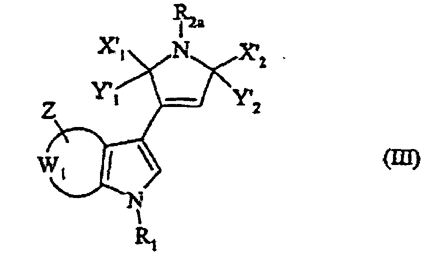 in der R₁, R₂ₐ, X'₁, Y'₁, X'₂, Y'₂, W₁ und Z die oben angegebenen Bedeutungen besitzen,
welche Verbindung der Formel (III):
* *entweder* mit wäßriger Natriumhydroxidlösung behandelt und dann mit Chlorwasserstoffsäure versetzt wird zur Bildung der Verbindung der Formel (IV): 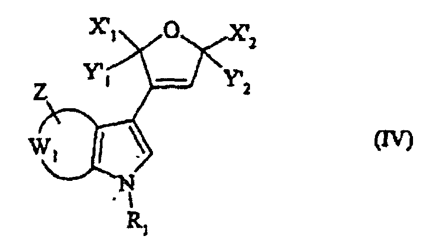 in der R₁, X'₁, Y'₁, X'₂, Y'₂, W₁ und Z die oben angegebenen Bedeutungen besitzen, welche Verbindung der Formel (IV) mit einer Verbindung der Formel (V) behandelt wird: 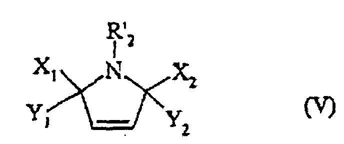 in der R'₂, X₁, Y₁, X₂ und Y₂ die bezüglich der Formel (I) angegebenen Bedeutungen besitzen, zur Bildung der Verbindung der Formeln (I/a) und (I/b), einem Sonderfällen der Verbindungen der Formel (I): 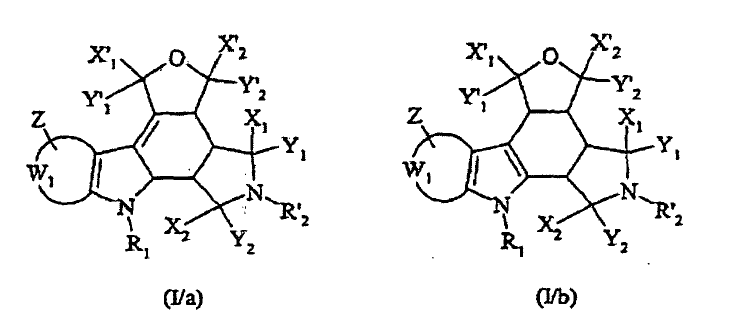 in denen R₁, R'₂, X₁, Y₁, X₂, Y₂, X'₁, Y'₁, X'₂, Y'₂, W₁ und Z die oben angegebenen Bedeutungen besitzen,
welche Verbindungen der Formeln (I/a) und/oder (I/b) gegebenenfalls der Einwirkung von Trifluoressigsäure unterworfen werden zur Bildung der Verbindung der Formel (I/c), einem Sonderfall der Verbindungen der Formel (I): 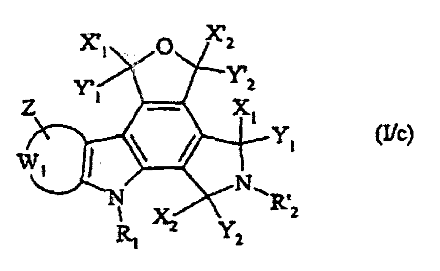 in der R₁, R'₂, X₁, Y₁, X₂, Y₂, X'₁, Y'₁, X'₂, Y'₂, W₁ und Z die oben angegebenen Bedeutungen besitzen,
wobei die Gesamtheit der Verbindungen der Formeln (I/a), (I/b) und (I/c) die Verbindungen der Formel (I/d) bildet: 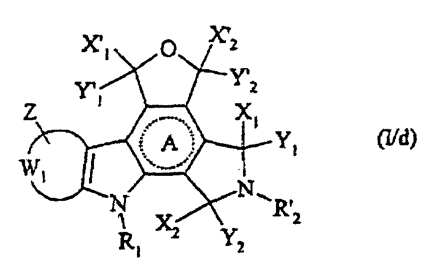 in der A, R₁, R'₂, X₁, Y₁, X₂, Y₂, X'₁, Y'₁, X'₂, Y'₂, W₁ und Z die oben angegebenen Bedeutungen besitzen,
welche Verbindung der Formel (I/d) gegebenenfalls der Einwirkung einer Verbindung der Formel (VII) unterworfen wird:
R_{2b} - NH₂ (VII)
in der R_{2b} die gleichen Bedeutungen besitzt wird für R₂ bezüglich der Formel (I) angegeben, mit Ausnahme der Definition des Wasserstoffatoms und der Methylgruppe, zur Bildung der Verbindungen der Formel (I/e), einem Sonderfall der Verbindungen der Formel (I): 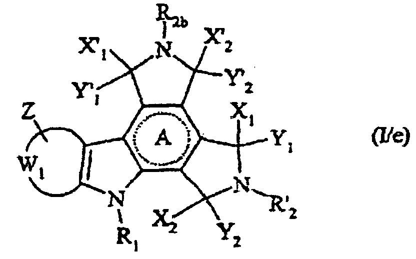 in der A, R₁, R'₂, R_{2b}, X₁, Y₁, X₂, Y₂, X'₁, Y'₁, X'₂, Y'₂, W₁ und Z die oben angegebenen Bedeutungen besitzen,
* *oder* nacheinander den gleichen Reaktionsbedingungen unterworfen wird wie die Verbindungen der Formel (IV), (I/a) und (I/b) zur Bildung der Verbindung der Formel (I/f), einem Sonderfall der Verbindungen der Formel (I): 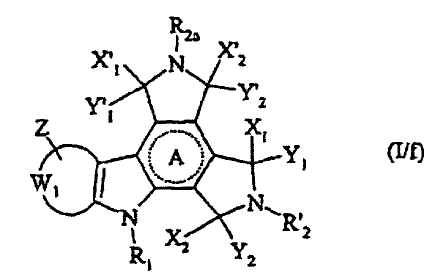 in der A, R₁, R'₂, R₂ₐ, X₁, Y₁, X₂, Y₂, X'₁, Y'₁, X'₂, Y'₂, W₁ und Z die oben angegebenen Bedeutungen besitzen,
wobei die Gesamtheit der Verbindungen (I/D), (I/e) und (I/f) die Verbindungen der Formel (I/g) bildet: 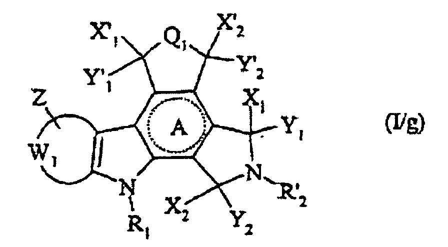 in der A, R₁, R'₂, Q₁, X₁, Y₁, X₂, Y₂, X'₁, Y'₁, X'₂, Y'₂, W₁ und Z die oben angegebenen Bedeutungen besitzen,
welche Verbindung der Formel (I/g) gegebenenfalls dann, wenn R'₂ ein Wasserstoffatom oder eine Methylgruppe bedeutet, nacheinander den gleichen Reaktionsbedingungen unterworfen wird wie die Verbindung der Formel (III) zur Bildung der Verbindung der Formel (I/i), einem Sonderfall der Verbindungen der Formel (I): 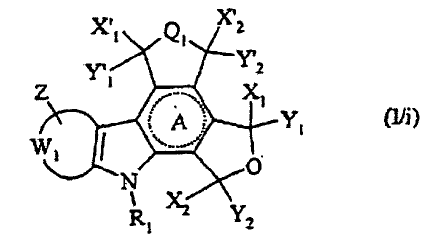 in der A, R₁, Q₁, X₁, Y₁, X₂, Y₂, X'₁, Y'₁, X'₂, Y'₂, W₁ und Z die oben angegebenen Bedeutungen besitzen,
welche Verbindung der Formel (I/i) gegebenenfalls der Einwirkung einer Verbindung (VIII) unterworfen wird:
R'_{2b} - NH₂ (VIII)
in der R'_{2b} die gleichen Bedeutungen besitzt wie R'₂ bezüglich der Formel (I), mit Ausnahme der Definition des Wasserstoffatoms und der Methylgruppe, zur Bildung der Verbindungen der Formel (I/j), einem Sonderfall der Verbindungen der Formel (I): 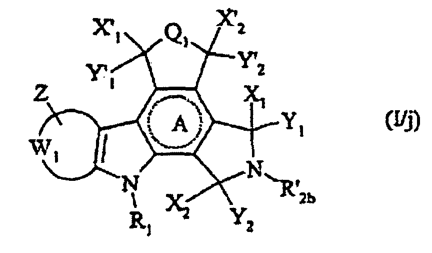 in der A, R₁, R'_{2b}, Q₁, X₁, Y₁, X₂, Y₂, X'₁, Y'₁, X'₂, Y'₂, W₁ und Z die oben angegebenen Bedeutungen besitzen,
welche Verbindungen der Formeln (I/a) bis (I/j), welche die Gesamtheit der Verbindungen der Formel (I) bilden, man gegebenenfalls mit Hilfe klassischer Reinigungsmethoden reinigt, welche gewünschtenfalls mit Hilfe einer klassischen Trennmethode in ihre verschiedenen Isomeren aufgetrennt werden können und bei denen man die Substituenten Rₐ, R_{b}, R_{c}, R_{d} und Rₑ mit Hilfe klassischer Methoden der organischen Chemie auf dem Gebiet der Zuckerchemie moduliert und welche man gewünschtenfalls in ihre Additionssalze mit einer pharmazeutisch annehmbaren Säure oder Base umwandelt.

20. Pharmazeutische Zubereitungen enthaltend als Wirkstoff mindestens eine Verbindung der Formel (I) nach einem der Ansprüche 1 bis 18 allein oder in Kombination mit einem oder mehreren inerten, nichttoxischen, pharmazeutisch annehmbaren Trägermaterialien oder Bindemitteln.

21. Pharmazeutische Zubereitungen nach Anspruch 20, nützlich als Arzneimittel bei der Behandlung von Krebs.

## Claims

1. Compounds of formula (I) : 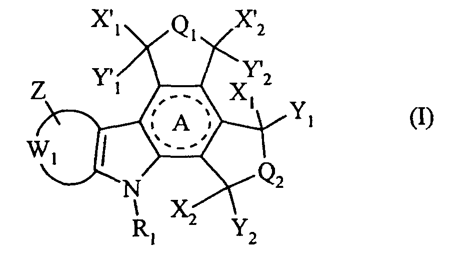 wherein :
• **A** represents a saturated or partially or fully unsaturated ring, wherein the unsaturation optionally confers an aromatic nature on the ring,
• **W**_{**1**}**,** together with the carbon atoms to which it is bonded, represents a phenyl group or a pyridyl group,
• **Z** represents one or more identical or different groups of formula U-V wherein :
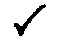 U represents a single bond, a linear or branched (C₁-C₆)alkylene chain or a linear or branched (C₂-C₆)alkenyl chain, optionally substituted by one or more identical or different groups selected from halogen and hydroxy, and/or optionally containing one or more unsaturated bonds,
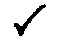 V represents a group selected from a hydrogen atom, a halogen atom and the groups cyano, nitro, azido, linear or branched (C₁-C₆)alkyl, aryl, aryl-(C₁-C₆)alkyl in which the alkyl moiety may be linear or branched, hydroxy, linear or branched (C₁-C₆)alkoxy, aryloxy, aryl-(C₁-C₆)alkoxy in which the alkoxy moiety may be linear or branched, formyl, carboxy, aminocarbonyl, NR₃R₄, -C(O)-T₁, -C(O)-NR₃-T_{1,} -NR₃-C(O)-T₁, -O-C(O)-T₁, -C(O)-O-T₁, -NR₃-T₂-NR₃R₄, -NR₃-T₂-OR₃, -NR₃-T₂-CO₂R₃, -O-T'₂-NR₃R₄, -O-T'₂-OR₃, -O-T'₂-CO₂R₃ and -S(O)ₜ-R₃,
wherein :
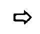 R₃ and R₄, which may be indentical or different, each represents a group selected from a hydrogen atom and the groups linear or branched (C₁-C₆)alkyl, aryl, and aryl-(C₁-C₆)alkyl in which the alkyl moiety may be linear or branched, or R₃+R₄, with the nitrogen atom carrying them, together form a saturated monocyclic or bicyclic heterocycle that has from 5 to 10 atoms, optionally contains in the ring system a second hetero atom selected from oxygen and nitrogen, and is optionally substituted by a group selected from linear or branched (C₁-C₆)alkyl, aryl, aryl-(C₁-C₆)alkyl in which the alkyl moiety may be linear or branched, hydroxy, linear or branched (C₁-C₆)alkoxy, amino, linear or branched mono-(C₁-C₆)alkylamino, and di(C₁-C₆)alkylamino in which the alkyl moieties may be linear or branched,
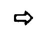 T₁ represents a group selected from linear or branched (C₁-C₆)alkyl that is optionally substituted by a group selected from -OR₃, -NR₃R₄, -CO₂R₃, -C(O)R₃ and -C(O)NR₃R₄ wherein R₃ and R₄ are as defined hereinbefore; aryl, and aryl-(C₁-C₆)alkyl in which the alkyl moiety may be linear or branched; or T₁ represents a linear or branched (C₂-C₆)alkenyl chain optionally substituted by a group selected from -OR₃, -NR₃R₄, -CO₂R₃, -C(O)R₃ and -C(O)NR₃R₄ wherein R₃ and R₄ are as defined hereinbefore,
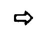 T₂ represents a linear or branched (C₁-C₆)alkylene chain,
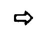 T'₂ represents a linear or branched (C₁-C₆)alkylene chain optionally substituted by one or more hydroxy groups,
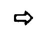 t represents an integer of from 0 to 2 inclusive,
or Z represents a methylenedioxy group or an ethylenedioxy group,
• **Q**_{**1**} represents a group selected from an oxygen atom and an NR₂ group wherein R₂ represents a group selected from a hydrogen atom and the groups linear or branched (C₁-C₆)alkyl, aryl, aryl-(C₁-C₆)alkyl in which the alkyl moiety may be linear or branched, cycloalkyl, cycloalkyl-(C₁-C₆)alkyl in which the alkyl moiety may be linear or branched, -OR₃, -NR₃R₄, -O-T₂-NR₃R₄, -NR₃-T₂-NR₃R₄, linear or branched (C₁-C₆)hydroxyalkylamino, di((C₁-C₆)hydroxyalkyl)amino in which the alkyl moieties may be linear or branched, -C(O)-R₃ and -NH-C(O)-R₃; or R₂ represents a linear or branched (C₁-C₆)alkylene chain optionally substituted by one or more identical or different groups selected from halogen atoms and the groups cyano, nitro, -OR₃, -NR₃R₄, -CO₂R₃, -C(O)R₃, linear or branched (C₁-C₆)hydroxyalkylamino, di((C₁-C₆)hydroxyalkyl)amino in which the alkyl moieties may be linear or branched, and -C(O)-NHR₃, the groups R₃, R₄ and T₂ being as defined hereinbefore,
• **Q**_{**2**} represents a group selected from an oxygen atom and an NR'₂ group wherein R'₂ represents a group selected from a hydrogen atom and the groups linear or branched (C₁-C₆)alkyl, aryl, aryl-(C₁-C₆)alkyl in which the alkyl moiety may be linear or branched, cycloalkyl, cycloalkyl-(C₁-C₆)alkyl in which the alkyl moiety may be linear or branched, -OR₃, -NR₃R₄, -O-T₂-NR₃R₄, -NR₃-T₂-NR₃R₄, linear or branched (C₁-C₆)hydroxyalkylamino, di((C₁-C₆)hydroxyalkyl)amino in which the alkyl moieties may be linear or branched, -C(O)-R₃ and -NH-C(O)-R₃; or R'₂ represents a linear or branched (C₁-C₆)alkylene chain substituted by one or more identical or different groups selected from halogen atoms and the groups cyano, nitro, -OR₃, -NR₃R₄, -CO₂R₃, -C(O)R₃, linear or branched (C₁-C₆)hydroxyalkylamino, di((C₁-C₆)-hydroxyalkyl)amino in which the alkyl moieties may be linear or branched, and -C(O)-NHR₃, the groups R₃, R₄ and T₂ being as defined hereinbefore,
• **X**_{**1**} represents a group selected from a hydrogen atom and the groups hydroxy, linear or branched (C₁-C₆)alkoxy, mercapto, and linear or branched (C₁-C₆)alkylthio,
• **Y**_{**1**} represents a hydrogen atom, or
• **X**_{**1**} and **Y**_{**1**}**,** with the carbon atom carrying them, together form a carbonyl or thiocarbonyl group,
• **X**_{**2**} represents a group selected from a hydrogen atom and the groups hydroxy, linear or branched (C₁-C₆)alkoxy, mercapto and linear or branched (C₁-C₆)alkylthio,
• **Y**_{**2**} represents a hydrogen atom, or
• **X**_{**2**} and **Y**_{**2**}**,** with the carbon atom carrying them, together form a carbonyl or thiocarbonyl group,
• **X'**_{**1**} represents a group selected from a hydrogen atom and the groups hydroxy, linear or branched (C₁-C₆)alkoxy, mercapto and linear or branched (C₁-C₆)alkylthio,
• **Y'**_{**1**} represents a hydrogen atom, or
• **X'**_{**1**} and **Y'**_{**1**}**,** with the carbon atom carrying them, together form a carbonyl or thiocarbonyl group,
• **X'**_{**2**} represents a group selected from a hydrogen atom and the groups hydroxy, linear or branched (C₁-C₆)alkoxy, mercapto and linear or branched (C₁-C₆)alkylthio,
• **Y'**_{**2**} represents a hydrogen atom, or
• **X'**_{**2**} and **Y'**_{**2**}**,** with the carbon atom carrying them, together form a carbonyl or thiocarbonyl group,
• **R**_{**1**} represents a group selected from a hydrogen atom and a linear or branched (C₁-C₆)alkyl group that is optionally substituted by one or more groups hydroxy, lineal or branched (C₁-C₆)alkoxy, linear or branched (C₁-C₆)hydroxyalkoxy and NR₃R₄, the groups R₃ and R₄ being as defined hereinbefore; or R₁ represents a group of formula (a) : 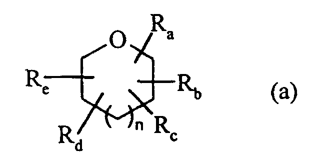
wherein :
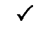 **R**_{**a**}**, R**_{**b**}**, R**_{**c**} and **R**_{**d**}**,** which may be identical or different, each represents, independently of the others, a bond or a group selected from a hydrogen atom, a halogen atom, and the groups hydroxy, linear or branched (C₁-C₆)alkoxy, aryloxy, aryl-(C₁-C₆)alkoxy in which the alkoxy moiety may be linear or branched, linear,or branched (C₁-C₆)alkyl, aryl-(C₁-C₆)alkyl in which the alkyl moiety may be linear or branched, aryl, -NR₃R₄ wherein R₃ and R₄ are as defined hereinbefore, azido, -N=NR₃ (wherein R₃ is as defined hereinbefore), and -O-C(O)-R₅ wherein R₅ represents a linear or branched (C₁-C₆)alkyl group (optionally substituted by one or more groups selected from halogen, hydroxy, amino, linear or branched (C₁-C₆)-alkylamino, and di(C₁-C₆)alkylamino in which the alkyl moieties may be linear or branched); or R₅ represents aryl, aryl-(C₁-C₆)alkyl in which the alkyl moiety may be linear or branched, cycloalkyl or heterocycloalkyl,
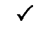 **R**_{**e**} represents a methylene group (H₂C=) or a group of formula -U₁-Rₐ wherein U₁ represents a single bond or a methylene group and Rₐ is as defined hereinbefore,
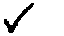 **n** is 0 or 1,
it being understood that the group of formula (a) is bonded to the nitrogen atom by Rₐ, R_{b}, R_{c}, R_{d} or Rₑ,
their enantiomers, diastereoisomers, and also addition salts thereof with a pharmaceutically acceptable acid or base, with the proviso that the compounds of formula (I) are other than the following compounds:
- 3b,6a,6b,7-tetrahydro-1*H*-dipyrrolo[3,4-a:3,4-c]carbazole-1,3,4,6-(2*H*,3a*H*,5*H*)-tetrone;
- 5-ethyl-3b,6a,6b,7-tetrahydro-1*H*-dipyrrolo[3,4-a:3,4-c]carbazole-1,3,4,6-(2*H*,3a*H*,5*H*)-tetrone;
- 3b,6a,7,11c-tetrahydro-1*H*-dipyrrolo[3,4-a:3,4-c]carbazole-1,3,4,6-(2*H*,3a*H*,5*H*)-tetrone;
- 3b,6a,6b,7-tetrahydrofuro[3,4-a]pyrrolo[3,4-c]carbazole-1,3,4,6-(2*H*,3a*H*,5*H*)-tetrone;
wherein aryl is understood to mean a phenyl, naphthyl, dihydronaphthyl, tetrahydronaphthyl, indenyl or indanyl group, each of those groups optionally being substituted by one or more identical or different groups selected from halogen, linear or branched (C₁-C₆)alkyl, linear or branched (C₁-C₆)trihaloalkyl, hydroxy, linear or branched (C₁-C₆)alkoxy, and NR₄R₄, R₃ and R₄ having the same definitions as hereinbefore.

2. Compounds of formula (I) according to claim 1, **characterised in that** X₁ and Y₁, with the carbon atom carrying them, together form a carbonyl group, X₂ and Y₂, with the carbon atom carrying them, together form a carbonyl group, X'₁ and Y'₁, with the carbon atom carrying them, together form a carbonyl group and X'₂ and Y'₂, with the carbon atom carrying them, together form a carbonyl group, their enantiomers, diastereoisomers, and also addition salts thereof with a pharmaceutically acceptable acid or base.

3. Compounds of formula (I) according to claim 1 or claim 2, **characterised in that** Q₁ represents an -NR₂ group wherein R₂ is as defined for formula (I), their enantiomers, diastereoisomers, and also addition salts thereof with a pharmaceutically acceptable acid or base.

4. Compounds of formula (I) according to any one of claims 1 to 3, **characterised in that** Q₂ represents an -NR'₂ group wherein R'₂ is as defined for formula (I), their enantiomers, diastereoisomers, and also addition salts thereof with a pharmaceutically acceptable acid or base.

5. Compounds of formula (I) according to any one of claims 1 to 4, **characterised in that** they represent compounds of formula (IA) : 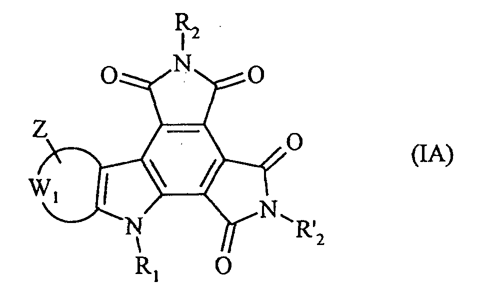 wherein R₁, R₂, R'₂, W₁ and Z are as defined for formula (I), their enantiomers, diastereoisomers, and also addition salts thereof with a pharmaceutically acceptable acid or base.

6. Compounds of formula (I) according to any one of claims 1 to 5, **characterised in that** they represent compounds of formula (IB) 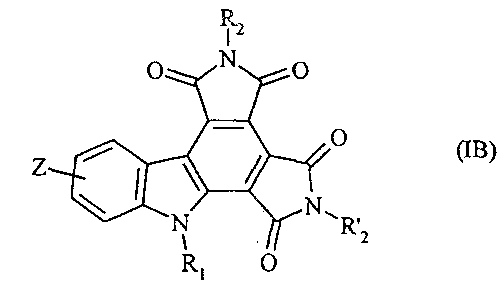 wherein R₁, R₂, R'₂ and Z are as defined for formula (I), their enantiomers, diastereoisomers, and also addition salts thereof with a pharmaceutically acceptable acid or base.

7. Compounds of formula (I) according to any one of claims 1 to 5, **characterised in that** they represent compounds of formula (IC) : 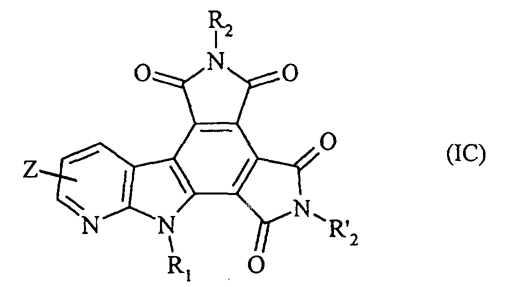 wherein R₁, R₂, R'₂ and Z are as defined for formula (I), their enantiomers, diastereoisomers, and also addition salts thereof with a pharmaceutically acceptable acid or base.

8. Compounds of formula (I) according to any one of claims 1 to 4, **characterised in that** they represent compounds of formula (ID) : 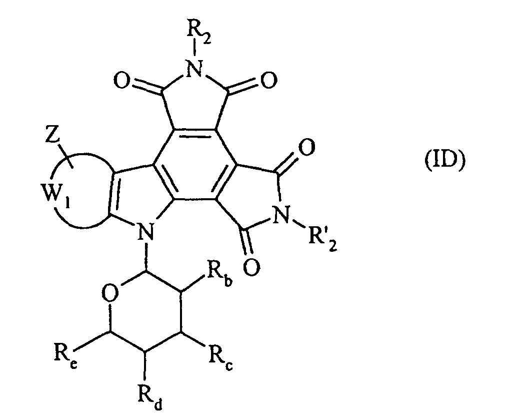 wherein R₂, R'₂, W₁, Z, R_{b}, R_{c}, R_{d} and Rₑ are as defined for formula (I), their enantiomers, diastereoisomers, and also addition salts thereof with a pharmaceutically acceptable acid or base.

9. Compounds of formula (I) according to any one of claims I to 4 and 8, **characterised in that** they represent compounds of formula (IE) : 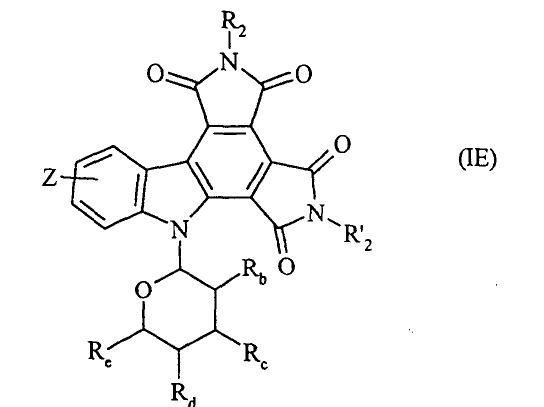 wherein R₂, R'₂, Z, R_{b}, R_{c}, R_{d} and Rₑ are as defined for formula (I), their enantiomers, diastereoisomers, and also addition salts thereof with a pharmaceutically acceptable acid or base.

10. Compounds of formula (I) according to any one of claims 1 to 4 and 8, **characterised in that** they represent compounds of formula (IF) : 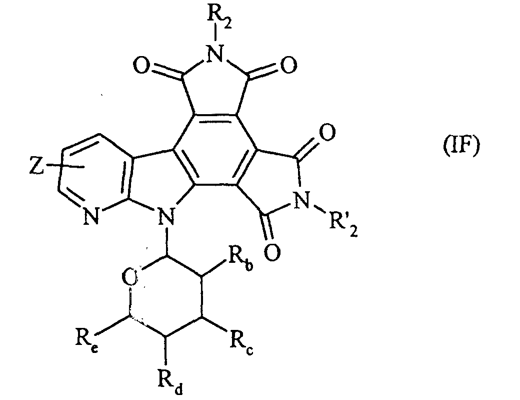 wherein R₂, R'₂, Z, R_{b}, R_{c}, R_{d} and Rₑ are as defined for formula (I), their enantiomers, diastereoisomers, and also addition salts thereof with a pharmaceutically acceptable acid or base.

11. Compounds of formula (I) according to any one of claims 1 to 10, **characterised in that** Z represents a group of formula U-V wherein U represents a single bond and V represents a group selected from a hydrogen atom, a halogen atom and the groups nitro, linear or branched (C₁-C₆)alkyl, hydroxy, linear or branched (C₁-C₆)alkoxy, aryl-(C₁-C₆)alkoxy in which the alkoxy moiety may be linear or branched, and NR₃R₄ wherein R₃ and R₄ each represents a hydrogen atom, their enantiomers, diastereoisomers, and also addition salts thereof with a pharmaceutically acceptable acid or base.

12. Compounds of formula (I) according to any one of claims 1 to 11, **characterised in that** Z represents a group of formula U-V wherein U represents a single bond and V represents a group selected from a hydrogen atom, a halogen atom and the groups hydroxy and aryl-(C₁-C₆)alkoxy in which the alkoxy moiety may be linear or branched, their enantiomers, diastereoisomers, and also addition salts thereof with a pharmaceutically acceptable acid or base.

13. Compounds of formula (I) according to any one of claims 1 to 12, **characterised in that** R₁ represents a hydrogen atom, a linear or branched (C₁-C₆)alkyl group or a group of formula (a) : 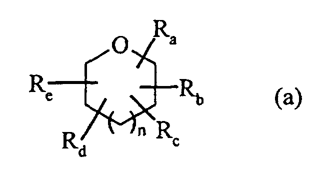 bonded to the nitrogen atom by Rₐ,
wherein :
• R_{b}, R_{c,} and R_{d} represent a hydroxy group, an aryl-(C₁-C₆)alkoxy group in which the alkoxy moiety may be linear or branched, or a group -O-C(O)-R₅ wherein R₅ represents a linear or branched (C₁-C₆)alkyl group,
• Rₑ represents a group of formula U₁-Rₐ wherein U₁ represents a methylene group and Rₐ has the same definitions as R_{b}, R_{c} and R_{d} and n is 0,
their enantiomers, diastereoisomers, and also addition salts thereof with a pharmaceutically acceptable acid or base.

14. Compounds of formula (I) according to any one of claims 1 to 13, **characterised in that** R₁ represents a hydrogen atom, their enantiomers, diastereoisomers, and also addition salts thereof with a pharmaceutically acceptable acid or base.

15. Compounds of formula (I) according to any one of claims 1 to 14, **characterised in that** R₂ represents a hydrogen atom, a linear or branched (C₁-C₆)alkyl group, OR₃, NR₃R₄, or a linear or branched (C₁-C₆)alkylene chain substituted by an OR₃ or NR₃R₄ group wherein R₃ and R₄ are as defined for formula (I), their enantiomers, diastereoisomers, and also addition salts thereof with a pharmaceutically acceptable acid or base.

16. Compounds of formula (I) according to any one of claims 1 to 15, **characterised in that** R₂ represents a hydrogen atom, a linear or branched (C₁-C₆)alkyl group, or a linear or branched (C₁-C₆)alkylene chain substituted by an NR₃R₄ group wherein R₃ and R₄ are as defined for formula I, their enantiomers, diastereoisomers, and also addition salts thereof with a pharmaceutically acceptable acid or base.

17. Compounds of formula (I) according to any one of claims 1 to 16, **characterised in that** R'₂ represents a hydrogen atom, a linear or branched (C₁-C₆)alkyl group, or a linear or branched (C₁-C₆)alkylene chain substituted by an NR₃R₄ group wherein R₃ and R₄ are as defined for formula (I), their enantiomers, diastereoisomers, and also addition salts thereof with a pharmaceutically acceptable acid or base.

18. Compounds of formula (I) according to claim 1 which are :
• 1*H*-dipyrrolo[3,4-a:3,4-c]carbazole-1,3,4,6-(2*H*,5*H*,7*H*)-tetrone,
• 2-methyl-1*H*-dipyrrolo[3,4-a:3,4-c]carbazole-1,3,4,6-(2*H*,5*H*,7*H*)-tetrone,
• 2,5-dimethyl-1*H*-dipyrrolo[3,4-a:3,4-c]carbazole-1,3,4,6-(2*H*,5*H*,7*H*)-tetrone,
• 2-[2-(diethylamino)ethyl]-5-methyl-1*H*-dipyrrolo[3,4-a:3,4-c]carbazole-1,3,4,6-(2*H*,5*H*,7*H*)-tetrone,
• 10-hydroxy-1*H*-dipyrrolo[3,4-a:3,4-c]carbazole-1,3,4,6-(2*H*,5*H*,7*H*)-tetrone.

19. Process for the preparation of compounds of formula (I), according to claim 1, **characterised in that** there is used as starting material a compound of formula (II) : 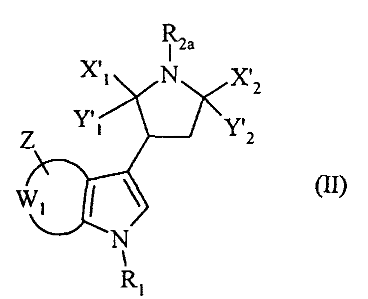 wherein R₂ₐ represents a hydrogen atom or a methyl group and R₁, X'₁, Y'₁, X'₂, Y'₂, W₁ and Z are as defined for formula (I),
which is treated with 2,3-dichloro-5,6-dicyano-1,4-benzoquinone to yield a compound of formula (III) : 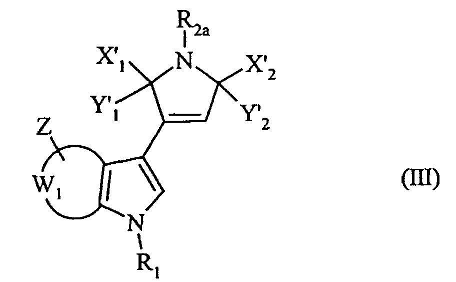 wherein R₁, R₂ₐ, X'₁, Y'₁, X'₂, Y'₂, W₁ and Z are as defined hereinbefore,
which compound of formula (III) is :
* *either* treated with aqueous sodium hydroxide solution and then placed in the presence of hydrochloric acid to yield a compound of formula (IV) : 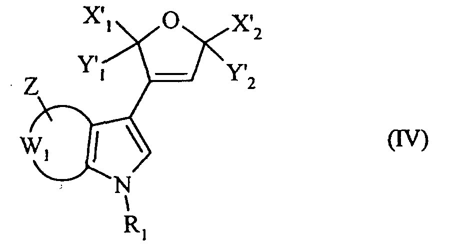 wherein R₁, X'₁, Y'₁, X'₂, Y'₂, W₁ and Z are as defined hereinbefore,
which compound of formula (IV) is treated with a compound of formula (V) : 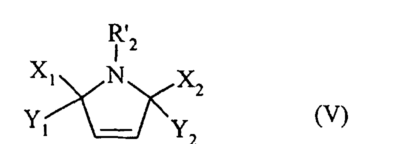 wherein R'₂, X₁, Y₁, X₂ and Y₂ are as defined for formula (I) to yield a compound of
formula (I/a) and (I/b), a particular case of the compounds of formula (I) : 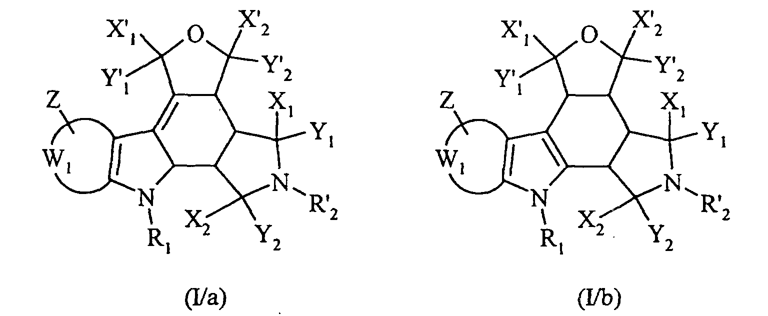 wherein R₁, R'₂, X₁, Y₁, X₂, Y₂, X'₁, Y'₁, X'₂, Y'₂, W₁ and Z are as defined hereinbefore,
which compound(s) of formula (I/a) and/or (I/b) is(are) optionally subjected to the action of trifluoroacetic acid to yield a compound of formula (I/c), a particular case of the compounds of formula (I) : 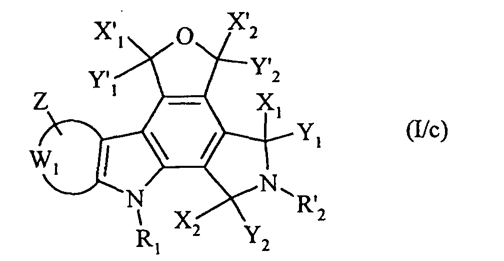 wherein R₁, R'₂, X₁, Y₁, X₂, Y₂, X'₁, Y'₁, X'₂, Y'₂, W₁ and Z are as defined hereinbefore,
the totality of the compounds of formulae (I/a), (I/b) and (I/c) constituting the compounds of formula (I/d) : 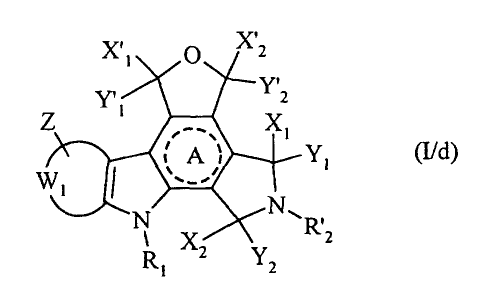 wherein A, R₁, R'₂, X₁, Y₁, X₂, Y₂, X'₁, Y'₁, X'₂, Y'₂, W₁ and Z are as defined hereinbefore,
which compound of formula (I/d) is optionally subjected to the action of a compound of formula (VII) :
R_{2b} - NH₂ (VII)
wherein R_{2b} has the same definition as R₂ in formula (I), with the exception of the definitions hydrogen atom and methyl group, to yield compounds of formula (I/e), a particular case of the compounds of formula (I): 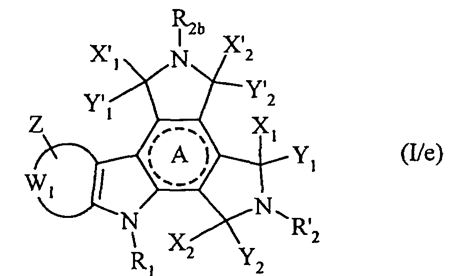 wherein A, R₁, R'₂, R_{2b}, X₁, Y₁, X₂, Y₂, X'₁, Y'₁, X'₂, Y'₂, W₁ and Z are as defined hereinbefore,
* *or* subjected in succession to the same reaction conditions as the compounds of formulae (IV), (I/a) and (I/b) to yield a compound of formula (I/f), a particular case of the compounds of formula (1) : 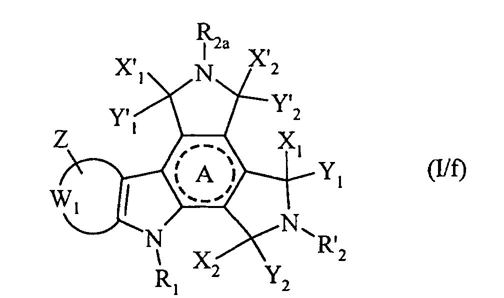 wherein A, R₁, R'₂, R₂ₐ, X₁, Y₁, X₂, Y₂, X'₁, Y'₁, X'₂, Y'₂, W₁ and Z are as defined hereinbefore,
the totality of the compounds (I/d), (I/e) and (I/f) constituting the compounds of formula (I/g) : 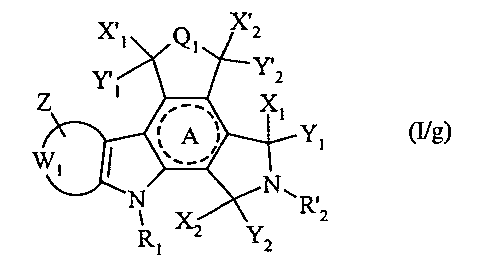 wherein A, R₁, R'₂, Q₁, X₁, Y₁, X₂, Y₂, X'₁, Y'₁, X'₂, Y'₂, W₁ and Z are as defined hereinbefore,
which compound of formula (I/g), when R'₂ represents a hydrogen atom or a methyl group, is optionally subjected in succession to the same reactions conditions as the compound of formulae (III) to yield a compound of formula (I/i), a particular case of the compounds of formula (I) : 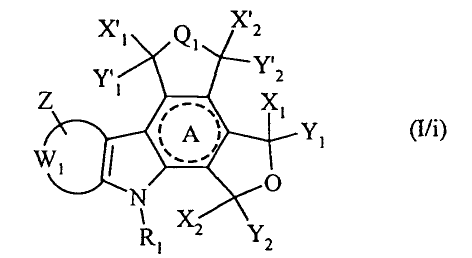 wherein A, R₁, Q₁, X₁, Y₁, X₂, Y₂, X'₁, Y'₁, X'₂, Y'₂, W₁ and Z are as defined hereinbefore,
which compound of formula (I/i) is optionally subjected to the action of a compound (VIII) :
R'_{2b} - NH₂ (VIII)
wherein R'_{2b} has the same definition as R'₂ in formula (I), with the exception of the definitions hydrogen atom and methyl group, to yield compounds of formula (I/j), a
particular case of the compounds of formula (I) : 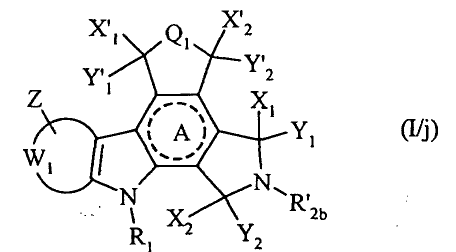 wherein A, R₁, R'_{2b}, Q₁, X₁, Y₁, X₂, Y₂, X'₁, Y'₁, X'₂, Y'₂, W₁ and Z are as defined hereinbefore,
the compounds of formulae (I/a) to (I/j) constituting the totality of the compounds of formula (I), which, if appropriate, are purified according to conventional purification techniques, may, if desired, be separated into their different isomers according to a conventional separation technique, the substituents Rₐ, R_{b}, R_{c}, R_{d} and Rₑ of which are modified according to conventional methods of organic synthesis used in the field of sugar chemistry, and which compounds, if desired, are converted into addition salts with a pharmaceutically acceptable acid or base.

20. Pharmaceutical compositions comprising as active ingredient at least one compound of formula (I) according to any one of claims 1 to 18, on its own or in combination with one or more pharmaceutically acceptable, inert, non-toxic excipients or carriers.

21. Pharmaceutical compositions according to claim 20 for use as medicaments in the treatment of cancers.
